(19) Europäisches Patentamt
European Patent Office
Office européen des brevets

(11) EP 1 317 426 B1

(12) EUROPÄISCHE PATENTSCHRIFT

(45) Veröffentlichungstag und Bekanntmachung des Hinweises auf die Patenterteilung:
**16.11.2005 Patentblatt 2005/46**

(21) Anmeldenummer: **01983480.3**

(22) Anmeldetag: **11.09.2001**

(51) Int Cl.[7]: **C07C 323/58**, C07C 323/61, A61K 31/198, A61P 25/00

(86) Internationale Anmeldenummer:
**PCT/EP2001/010488**

(87) Internationale Veröffentlichungsnummer:
**WO 2002/022568 (21.03.2002 Gazette 2002/12)**

(54) **BETA-THIO-AMINOSÄUREN**

BETA-THIO-AMINO ACIDS

ACIDES $G(B)-THIO-AMINES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **14.09.2000 DE 10045831**
**29.09.2000 DE 10049484**

(43) Veröffentlichungstag der Anmeldung:
**11.06.2003 Patentblatt 2003/24**

(73) Patentinhaber: **Grünenthal GmbH**
**52078 Aachen (DE)**

(72) Erfinder:
- **CHIZH, Boris**
  **Cambridge CB2 4NW (GB)**
- **GERLACH, Matthias**
  **63636 Brachttal (DE)**
- **HAURAND, Michael**
  **52078 Aachen (DE)**
- **PÜTZ, Claudia**
  **52349 Düren (DE)**
- **GAUBE, Gero**
  **52062 Aachen (DE)**
- **ENDERS, D.**
  **52078 Aachen (DE)**

(56) Entgegenhaltungen:
**WO-A-00/15611**
**WO-A-00/74705**
**WO-A-01/20336**
**GB-A- 2 336 587**
**US-A- 4 024 175**

- **DATABASE REGISTRY [Online] CHEMICAL ABSTRACTS SERVICE, COLUMBUS, OHIO, US; RN = 115310-95-7, XP002189463 & JP 06 059399 A (KONISHIROKU PHOTO IND.) 4. März 1994 (1994-03-04)**

EP 1 317 426 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft β-Thio-α-Aminosäuren, Verfahren zu deren Herstellung, Arzneimittel enthaltend diese Verbindungen und die Verwendung von Thioaminosäuren zur Herstellung von Arzneimitteln.

Das cyclische GABA Analoge Gabapentin ist ein klinisch erprobtes Antiepileptikum. Gabapentin zeigt zudem weitere interessante, medizinische relevante Eigenschaften, insbesondere als Analgetikum. Interessant sind deshalb neue Strukturklassen, die Affinität zur Gabapentin-Bindungsstelle aufweisen. Es besteht bei den genannten Indikationen weiterer Bedarf an Substanzen, die in ihren Eigenschaften Übereinstimmungen mit Gabapentin zeigen, beispielsweise in der analgetischen Wirkung.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Aus der JP 06059399, welche die Herstellung von photographischen Entwicklern betrifft, ist die Verbindung 3-Mercapto-3,4-dimethyl-5-oxo-norleucin bekannt.

In der US 4,024,175 wird die Herstellung von zyklischen Aminosäuren wie beispielsweise 1-Aminomethyl-1-cyclohexan-essigsäure und deren Verwendung als pharmakologische Wirkstoffe in Arzneimitteln offenbart.

Der WO 00/15611 sind Pyrrolidin-3-carboxylsäuren mit verzweigten Alkylketten, sowie deren Verwendung als pharmakologische Wirkstoffe in Arzneimitteln zu entnehmen.

Aus der GB 2 336 587 sind substituierte Tetralon-Derivate bekannt, die eine Affinität für GABA-Rezeptoren aufweisen, sowie deren Verwendung zur Herstellung von Arzneimitteln.

Die nachveröffentlichte WO 01/20336 beschreibt ein Verfahren für das Screening von Liganden mit Affinität zu der $\alpha_2\delta$-1 Untereinheit eines spannungsabhängigen Kalziumkanals unter Verwendung von radioaktiv markierten Aminosäuren.

Aus der ebenfalls nachveröffentlichten WO 00/74705 sind Bis[1,2-Aminothiole] und Bis[1,3-Aminothiole] sowie deren Eignung zur Herstellung von Arzneimitteln bekannt.

Aufgabe der Erfindung war es daher, Strukturen, vorzugsweise neue Strukturen, aufzufinden, die Affinität zur Gabapentin-Bindungsstelle und/oder entsprechende physiologische Wirksamkeiten, beispielsweise in Hinblick auf Analgesie, aber auch andere GBP-Indikationen, aufweisen, aufzufinden.

Gegenstand der Erfindung ist daher die Verwendung einer β-Thio-α-aminosäure der allgemeinen Formel I,

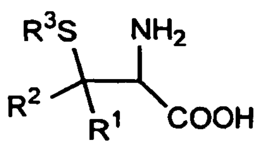

I

, worin

$R^1$ und $R^2$ jeweils unabhängig voneinander ausgewählt sind aus H; $C_{1-10}$-Alkyl, verzweigt oder unverzweigt, gesättigt oder ungesättigt, unsubstituiert oder ein- oder mehrfach substituiert; Benzyl, Aryl, $C_{3-8}$-Cycloalkyl oder Heteroaryl, jeweils unsubstituiert oder ein- oder mehrfach substituiert; oder

$R^1$ und $R^2$ zusammen einen $(CH_2)_{3-6}$-Ring bilden, gesättigt oder ungesättigt, substituiert oder unsubstituiert, in dem 0-2 C-Atome durch S, O oder $NR^4$, ersetzt sein können,

mit $R^4$ ausgewählt aus: H; $C_{1-10}$-Alkyl, gesättigt oder ungesättigt, verzweigt oder unverzweigt, einfach oder mehrfach substituiert oder unsubstituiert;

$R^3$ausgewählt ist aus H; $C_{1-10}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes $C_{1-3}$-Alkyl gebundenem Aryl, $C_{3-8}$-Cycloalkyl oder Heteroaryl, jeweils unsubstituiert oder

einfach oder mehrfach substituiert;
in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen;
mit Ausnahme von Verbindungen, bei denen $R^1$, $R^2$ und $R^3$ gleichzeitig H sind oder $R^1$ und $R^2$ gleichzeitig $CH_3$ sind und $R^3$ Wasserstoff entspricht,
zur Herstellung eines Arzneimittels zur Behandlung von Schmerz, insbesondere von neuropathischem, chronischem oder akutem Schmerz, von Epilepsie und/oder von Migräne
oder
zur Herstellung eines Arzneimittels zur Behandlung von Hyperalgesie und Allodynie, insbesondere thermischer Hyperalgesie, mechanischer Hyperalgesie und Allodynie und Kälte Allodynie, oder von inflammatorischem oder postoperativem Schmerz
oder
zur Herstellung eines Arzneimittels zur Behandlung von Hitzewallungen, Beschwerden in der Postmenopause , Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

Diese Substanzen binden an die Gabapentin-Bindungsstelle und zeigen eine ausgeprägte analgetische Wirkung.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7-oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl unter dem Begriff substituiert im Sinne dieser Erfindung die Substitution eines Wasserstoffrestes durch F, Cl, Br, I, $NH_2$, SH oder OH, wobei unter "mehrfach substituiert" Resten zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, $-CH_2-CH_2-$, $-CH_2-CH_2-CH_2-$ und $-CH_2-CH_2-CH_2-CH_2-$ zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring

verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{23}$, $OR^{23}$ einem Halogen, vorzugsweise F und/oder Cl, einem $CF_3$, einem CN, einem $NO_2$, einem $NR^{24}R^{25}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{23}$ für H, einen $C_{1-10}$-Alkyl-, vorzugsweise einen $C_{1-6}$-Alkyl-, einen Aryl- oder Heteroaryl- oder für einen über eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste $R^{24}$ und $R^{25}$, gleich oder verschieden, für H, einen $C_{1-10}$-Alkyl-, vorzugsweise einen $C_{1-6}$-Alkyl-, einen Aryl-, einen Heteroaryl- oder einen über eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste $R^{24}$ und $R^{25}$ bedeuten zusammen $CH_2CH_2OCH_2CH_2$, $CH_2CH_2NR^{26}CH_2CH_2$ oder $(CH_2)_{3-6}$, und
der Rest $R^{26}$ für H, einen $C_{1-10}$-Alkyl-, vorzugsweise einen $C_{1-6}$-Alkyl-, einen Aryl-, oder Heteroaryl- Rest oder für einen über eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Alle hier vorangehend aufgeführten und für die Verwendung definierten Substanzen verdrängen Gabapentin von seiner - auch in der Wissenschaft bisher noch unbekannten - Bindungsstelle. Das impliziert aber, daß die erfindungsgemäßen Substanzen an der gleichen Bindungsstelle binden und über sie physiologisch wirken werden, vermutlich mit dem gleichen Wirkungsprofil wie Gabapentin. Daß diese Annahme der gleichen Wirkung bei gleicher Bindungsstelle auch zutrifft, wird durch die analgetische Wirkung bewiesen. So verdrängen die erfindungsgemäßen Verbindungen nicht nur Gabapentin von seiner Bindungsstelle sondern wirken auch - wie Gabapentin - deutlich analgetisch. Entsprechend ist der Gegenstand der Erfindung die Verwendung der genannten und definierten Thioaminosäuren in den vorangehend genannten Indikationen, in denen Gabapentin wirkt, also insbesondere in der Schmerztherapie, bei Epilepsie oder Migräne, aber speziell auch im neuropathischen Schmerz also Hyperalgesie und Allodynie und den anderen Gabapentin Indikationen.

Gabapentin ist ein bekanntes Antiepileptikum mit antikonvulsiver Wirkung. Neben dieser wird Gabapentin von auch in verschiedenen anderen Indikation eingesetzt, unter anderem von behandelnden Ärzten bei Migräne und bipolaren Störungen sowie Hitzewallungen (z.B. in der Postmenopause) verschrieben (M. Schrope, Modern Drug Discovery, September 2000, S. 11). Andere Indikationenen, in denen Gabapentin ein therapeutisches Potential zeigt, wurden während der Humanstudien und im klinischen Gebrauch identifiziert (J.S. Bryans, D.J. Wustrow; "3-Substituted GABA Analogs with Central Nervous System Activity: A Review" in Med. Res. Rev. (1999), S. 149-177). In diesem Übersichtsartikel wird detailliert die Wirkung von Gabapentin aufgelistet. So ist Gabapentin wirksam in der Behandlung chronischer Schmerzen und Verhaltensstörungen. Insbesondere sind aufgeführt: Antikonvulsive und antiepileptische

Wirkungen, der Einsatz gegen chronischen, neuropathischen Schmerz, insbesondere thermische Hyperalgesie, mechanische Allodynie, Kälte Allodynie. Weiter wirkt es gegen durch Nervenschädigungen ausgelöste Neuropathie, insbesondere eben neuropathischen Schmerz, wie auch inflammatorischen und postoperativen Schmerz erfolgreich. Gabapentin ist auch erfolgreich bei antipsychotischen Effekten insbesondere als Anxiolytikum. Weitere überprüfte Indikationen umfassen: Amyotropische Laterale Sklerose (ALS), Reflex Sympasthetic Dystrophy (RSD), Spastische Lähmung, Restless Leg Syndrom, Behandlung von Symptomen und Schmerz aufgrund von Multipler Sklerose, erworbener Nystagmus, Behandlung der Symptome der Parkinsonschen Krankheit, der schmerzvollen diabetischen Neuropathie und psychatrischer Störungen, z.B. bipolare Störungen, Stimmungsschwankungen, manisches Verhalten. Weiter erfolgreich war der Einsatz von Gabapentin bei erythromelalgischem Schmerz, postpoliomyelitisem Schmerz, trigeminaler Neuralgie und postherpetischer Neuralgie (Bryans und Wustrow (1999), a.a.O.). Allgemein bekannt und auch dem genannten Übersichtsartikel anhand der Beispiele zu entnehmen ist auch die allgemeine Wirksamkeit in neurodegenerativen Erkrankungen. Solche Neurodegenerativen Erkrankungen sind z.B. Morbus Alzheimer, Morbus Huntington, Morbus Parkinson und Epilepsie. Bekannt ist auch die Wirksamkeit von Gabapentin bei gastrointestinalen Schädigungen.

In einer bevorzugten Ausführungsform wird in diesen Indikationen eine Thioaminosäure gemäß Formel verwendet, worin

$R^1$ und $R^2$ jeweils unabhängig voneinander ausgewählt sind aus $C_{1-10}$-Alkyl, verzweigt oder unverzweigt, gesättigt oder ungesättigt, unsubstituiert oder ein- oder mehrfach substituiert; Benzyl, Aryl, $C_{3-8}$-Cycloalkyl oder Heteroaryl, jeweils unsubstituiert oder ein- oder mehrfach substituiert;

oder

$R^1$ und $R^2$ zusammen einen $(CH_2)_{3-6}$-Ring bilden, gesättigt oder ungesättigt, substituiert oder unsubstituiert, in dem 0-2 C-Atome durch S, O oder $NR^4$, ersetzt sein können.

In einer weiteren bevorzugten Ausführungsform wird in diesen Indikationen eine Thioaminosäure gemäß Formel I verwendet; worin

$R^1$ und $R^2$ jeweils unabhängig voneinander ausgewählt sind aus H; $C_{1-10}$-Alkyl, verzweigt oder unverzweigt, gesättigt oder ungesättigt, unsubstituiert oder ein- oder mehrfach substituiert; Phenyl oder Thiophenyl, jeweils unsubstituiert oder einfach (vorzugsweise mit $OCH_3$, $CH_3$, OH, SH, $CF_3$, F, Cl, Br oder I) substituiert; oder $C_{3-8}$-Cycloalkyl, unsubstituiert oder substituiert,

oder

$R^1$ und $R^2$ zusammen einen $(CH_2)_{3-6}$-Ring bilden, substituiert oder unsubstituiert, in dem 0-1 C-Atome durch S, O oder $NR_4$, ersetzt sein können,

vorzugsweise

einer der Reste $R^1$ und $R^2$ $C_{1-2}$-Alkyl, insbesondere Methyl oder Ethyl, jeweils unsubstituiert oder ein- oder mehrfach substituiert; oder Phenyl, Thiophenyl, jeweils unsubstituiert oder einfach (vorzugsweise mit $OCH_3$, $CH_3$, OH, SH, $CF_3$, F, Cl, Br oder I) substituiert; oder $C_{3-8}$-Cycloalkyl unsubstituiert oder einfach substituiert; bedeutet und der andere der Reste $R^1$ und $R^2$ $C_{2-10}$-Alkyl, insbesondere Ethyl, n-Propyl, i-Propyl, n-Butyl, i-Butyl, tert.-Butyl, Pentyl, Hexyl, Heptyl oder Octyl, verzweigt oder unverzweigt, gesättigt oder ungesättigt, unsubstituiert oder ein- oder mehrfach substituiert; oder Phenyl bzw. Thiophenyl, jeweils unsubstituiert oder einfach (vorzugsweise mit $OCH_3$, $CH_3$, OH, SH, $CF_3$, F, Cl, Br oder I) substituiert; oder $C_{3-8}$-Cycloalkyl, insbesondere Cyclopropyl, Cyclobutyl, Cyclopentyl, Cyclohexyl oder Cycloheptyl, jeweils unsubstituiert oder einfach substituiert; bedeutet,

oder

$R^1$ und $R^2$ zusammen Cyclopropyl, Cyclobutyl, Cyclopentyl, Cyclohexyl oder Cycloheptyl, insbesondere Cyclopropyl, Cyclobutyl oder Cyclopentyl, bilden, jeweils unsubstituiert oder einfach substituiert, wobei gegebenenfalls ein C-Atom im Ring durch S ersetzt ist.

In einer bevorzugten Ausführungsform wird in diesen Indikationen eine Thioaminosäure gemäß Formel I verwendet, worin

$R_3$ ausgewählt ist aus H; $C_{1-6}$-Alkyl, gesättigt oder ungesättigt, verzweigt oder unverzweigt, einfach oder mehrfach substituiert oder unsubstituiert; Phenyl oder Thiophenyl, unsubstituiert oder einfach (vorzugsweise mit $OCH_3$, $CH_3$, OH, SH, $CF_3$, F, Cl, Br oder I) substituiert; oder über gesättigtes $CH_3$-gebundenem Phenyl, unsubstituiert oder einfach (vorzugsweise mit $OCH_3$, $CH_3$, OH, SH, $CF_3$, F, Cl, Br oder I) substituiert;

vorzugsweise $R^3$ ausgewählt ist aus H; $C_{1-6}$-Alkyl, gesättigt, unverzweigt und unsubstituiert, insbesondere Methyl, Ethyl, Propyl, n-Propyl, i-Propyl, Butyl, n-Butyl, i-Butyl, tert.-Butyl, Pentyl oder Hexyl; Phenyl oder Thiophenyl, unsubstituiert oder einfach (vorzugsweise mit $OCH_3$, $CH_3$, OH, SH, $CF_3$, F, Cl, Br oder I) substituiert; oder über gesättigtes $CH_3$-gebundenem Phenyl, unsubstituiert oder einfach (vorzugsweise mit $OCH_3$, $CH_3$, OH, SH, $CF_3$, F, Cl, Br oder I) substituiert.

Es ist weiter bevorzugt, wenn für die erfindungsgemäße Verwendung weiter gilt, daß für die verwendete Thioaminosäure gemäß Formel I gilt, daß,

- wenn einer von $R^1$ öder $R^2$ Wasserstoff ist und $R^3$ Benzyl oder H ist, der andere von $R^1$ oder $R^2$ nicht Phenyl sein darf,
- wenn $R^1$ und $R^2$ zusammen Cyclopentyl bilden, $R^3$ nicht H sein darf,
- wenn einer von $R^1$ oder $R^2$ Wasserstoff und der andere von $R^1$ oder $R^2$ Phenyl ist, $R^3$ nicht substituiertes oder unsubstituiertes Benzyl sein darf oder
- wenn einer von $R^1$ oder $R^2$ Wasserstoff und der andere von $R^1$ oder $R^2$ Methyl ist, $R^3$ nicht H sein darf.

In einer weiteren bevorzugten Ausführungsform der Erfindung wird eine Thioaminosäure ausgewählt aus der folgenden Gruppe verwendet:

- 2-Amino-3-mercapto-3-methyl-pentansäure
- 2-Amino-3-mercapto-3-methyl-hexansäure
- 2-Amino-3-mercapto-3-methyl-heptansäure
- 2-Amino-3-mercapto-3-methyl-octansäure
- 2-Amino-3-mercapto-3-methyl-nonansäure
- 2-Amino-3-mercapto-3-methyl-decansäure
- 2-Amino-3-ethyl-3-mercapto-pentansäure
- Amino-(1 -mercapto-cyclopentyl)essigsäure
- Amino-3-ethyl-3-mercapto-hexansäure
- 2-Amino-3-mercapto-3-methyl-decansäure
- 2-Amino-3-mercapto-3-methyl-nonansäure
- 2-Amino-3-mercapto-3-methyl-octansäure
- 2-Amino-3-ethylsulfanyl-3-methyl-octansäure
- 2-Amino-3-benzylsulfanyl-3-methyl-octansäure
- 2-Amino-3-mercapto-3-propyl-3-hexansäure
- Amino-(1-mercapto-cycloheptyl)-essigsäure
- 2-Amino-3-mercapto-3-propyl-3-hexansäure
- Amino-(1-mercapto-cycloheptyl)-essigsäure
- 2-Amino-3-ethylsulfanyl-3-methyl-nonansäure
- 2-Amino-3-methyl-3-propylsulfanyl-nonansäure
- 2-Amino-3-hexylsulfanyl-3-methyl-nonansäure
- 2-Amino-3-benzylsulfanyl-3-methyl-nonansäure
- 2-Amino-3-benzylsulfanyl-3-methyl-decansäue
- 2-Amino-3-ethylsulfanyl-3-methyl-decansäure
- 2-Amino-3-cyclopropyl-3-(4-fluoro-phenyl)-3-mercapto-propansäure
- 2-Amino-3-cyclopropyl-3-mercapto-butansäure
- 2-Amino-3-cyclobutyl-3-mercapto-butansäure
- 2-Amino-3-cyclohexyl-3-mercapto-butansäure
- 2-Amino-3-mercapto-3-thiophen-2-yl-butansäure
- 2-Amino-3-ethyl-3-mercapto-heptansäure
- Amino-(1-mercapto-cyclohexyl)- ethansäure
- Amino-(1-mercapto-3-methyl-cyclohexyl)-ethansäure
- Amino-(1-mercapto-2-methyl-cyclohexyl)-ethansäure
- Amino-(1-mercapto-4-methyl-cyclohexyl)-ethansäure
- Amino-(4-mercapto-tetrahydro-thiopyran-4-yl)-ethansäure
- 2-Amino-3-mercapto-3,4-dimethyl-pentansäur
- 2-Amino-3-mercapto-3,4-dimethyl-hexansäure

in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen, vorzugsweise des Hydrochlorids.

Es ist weiter bevorzugt, wenn bei der erfindungsgemäßen Verwendung mindestens eine verwendete Thioaminosäure als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung sind β-Thio-α-aminosäuren der allgemeinen Formel I,

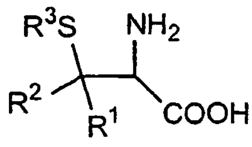

I

, worin

einer der Reste $R^1$ und $R^2$ $C_{1-6}$-Alkyl, gesättigt oder ungesättigt, verzweigt oder unverzweigt, einfach oder mehrfach substituiert oder unsubstituiert; bedeutet und der andere der Reste $R^1$ und $R^2$ $C_{3-10}$-Alkyl, gesättigt oder ungesättigt, verzweigt oder unverzweigt, einfach oder mehrfach substituiert oder unsubstituiert; oder Phenyl, Thiophenyl oder $C_{3-8}$-Cycloalkyl, jeweils unsubstituiert oder einfach oder mehrfach substituiert; bedeutet,

und

$R^3$ ausgewählt ist aus H; $C_{1-0}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes $C_{1-3}$-Alkyl gebundenem Aryl, $C_{3-8}$-Cycloalkyl oder Heteroaryl, jeweils unsubstituiert oder einfach oder mehrfach substituiert;

in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen.

Eine bevorzugte Ausführungsform der Erfindung ist eine erfindungsgemäße Thioaminosäure, worin

einer der Reste $R^1$ und $R^2$ $C_{1-2}$-Alkyl, einfach oder mehrfach substituiert oder unsubstituiert, insbesondere Methyl oder Ethyl; bedeutet und der andere der Reste $R^1$ und $R^2$ $C_{3-10}$-Alkyl, vorzugsweise $C_{3-8}$-Alkyl, gesättigt oder ungesättigt, verzweigt oder unverzweigt, einfach oder mehrfach substituiert oder unsubstituiert, insbesondere Propyl, n-Propyl, i-Propyl, Butyl, n-Butyl, i-Butyl, tert.-Butyl, Pentyl, Hexyl, Heptyl oder Octyl; oder Phenyl oder Thiophenyl, jeweils unsubstituiert oder einfach (vorzugsweise mit $OCH_3$, $CH_3$, OH, SH, $CF_3$, F, Cl, Br oder I) substituiert; oder Cyclopropyl, Cyclobutyl, Cyclopentyl, Cyclohexyl oder Cycloheptyl; bedeutet.

Eine bevorzugte Ausführungsform der Erfindung ist eine erfindungsgemäße Thioaminosäure, worin

$R_3$ ausgewählt ist aus H; $C_{1-6}$-Alkyl, gesättigt oder ungesättigt, verzweigt oder unverzweigt, einfach oder mehrfach substituiert oder unsubstituiert; Phenyl oder Thiophenyl, unsubstituiert oder einfach (vorzugsweise mit $OCH_3$, $CH_3$, OH, SH, $CF_3$, F, Cl, Br oder I) substituiert; oder über gesättigtes $CH_3$-gebundenem Phenyl, unsubstituiert oder einfach (vorzugsweise mit $OCH_3$, $CH_3$, OH, SH, $CF_3$, F, Cl, Br oder I) substituiert; vorzugsweise $R^3$ ausgewählt ist aus H; $C_{1-6}$-Alkyl, gesättigt, unverzweigt und unsubstituiert, insbesondere Methyl, Ethyl, Propyl, n-Propyl, i-Propyl, Butyl, n-Butyl, i-Butyl, tert.-Butyl, Pentyl oder Hexyl; Phenyl oder Thiophenyl, unsubstituiert oder einfach (vorzugsweise mit $OCH_3$, $CH_3$, OH, SH, $CF_3$, F, Cl, Br oder I) substituiert; oder über gesättigtes $CH_3$-gebundenem Phenyl, unsubstituiert oder einfach (vorzugsweise mit $OCH_3$, $CH_3$, OH, SH, $CF_3$, F, Cl, Br oder I) substituiert.

Bei einer sehr bevorzugten Ausführungsform der der Erfindung ist die erfindungsgemäße Thioaminosäure ausgewählt aus der folgenden Gruppe:

- 2-Amino-3-mercapto-3-methyl-hexansäure
- 2-Amino-3-mercapto-3-methyl-heptansäure
- 2-Amino-3-mercapto-3-methyl-octansäure
- 2-Amino-3-mercapto-3-methyl-nonansäure
- 2-Amino-3-mercapto-3-methyl-decansäure
- Amino-3-ethyl-3-mercapto-hexansäure
- 2-Amino-3-mercapto-3-methyl-decansäure
- 2-Amino-3-mercapto-3-methyl-nonansäure
- 2-Amino-3-mercapto-3-methyl-octansäure
- 2-Amino-3-ethylsulfanyl-3-methyl-octansäure
- 2-Amino-3-benzylsulfanyl-3-methyl-octansäure
- 2-Amino-3-mercapto-3-propyl-3-hexansäure
- Amino-(1-mercapto-cycloheptyl)-essigsäure
- 2-Amino-3-mercapto-3-propyl-3-hexansäure
- 2-Amino-3-ethylsulfanyl-3-methyl-nonansäure
- 2-Amino-3-methyl-3-propylsulfanyl-nonansäure

- 2-Amino-3-hexylsulfanyl-3-methyl-nonansäure
- 2-Amino-3-benzylsulfanyl-3-methyl-nonansäure
- 2-Amino-3-benzylsulfanyl-3-methyl-decansäue
- 2-Amino-3-ethylsulfanyl-3-methyl-decansäure
- 2-Amino-3-cyclopropyl-3-mercapto-butansäure
- 2-Amino-3-cyclobutyl-3-mercapto-butansäure
- 2-Amino-3-cyclohexyl-3-mercapto-butansäure
- 2-Amino-3-mercapto-3-thiophen-2-yl-butansäure
- 2-Amino-3-ethyl-3-mercapto-heptansäure
- 2-Amino-3-mercapto-3,4-dimethyl-pentansäure
- 2-Amino-3-mercapto-3,4-dimethyl-hexansäure

in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen, vorzugsweise des Hydrochlorids.

Die erfindungsgemäßen Substanzen sind toxikologisch unbedenklich, so daß sie sich als pharmazeutischer Wirkstoff in Arzneimitteln eignen. Ein weiterer Gegenstand der Erfindung sind daher Arzneimittel enthaltend wenigstens eine erfindungsgemäße Thioaminosäure, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Thioaminosäure gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Thioaminosäuren in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Thioaminosäuren verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens einer erfindungsgemäßen Thioaminosäure appliziert.

In einer bevorzugten Form des Arzneimittel liegt eine enthaltene erfindungsgemäße Thioaminosäure als reines Diastereomer und/oder Enantiomer, als Razemat oder als nicht-äquimolare oder äquimolare Mischung der Diastereomere und/oder Enantiomere vor.

Dabei kann es bevorzugt sein; wenn eine verwendete erfindungsgemäße Thioaminosäure als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung eines nichthumanen Säugetieres oder Menschen, das oder der eine Behandlung medizinisch relevanter Symptome benötigt, durch Verabreichung einer therapeutisch wiksamen Dosis einer oben genannten, vorzugsweise erfindungsgemäßen oder erfindungsgemäß verwendeten, Thioaminosäure oder eines erfindungsgemäßen Arzneimittels. Die Erfindung betrifft insbesondere entsprechende Verfahren zur Behandlung von Schmerz, insbesondere von neuropathischem, chronischem oder akutem Schmerz; Migräne, Hyperalgesie und Allodynie, insbesondere thermischer Hyperalgesie, mechanischer Hyperalgesie und Allodynie und Kälte Allodynie, oder von inflammatorischem oder postoperativem Schmerz; Epilepsie, Hitzewallungen, Beschwerden in der Postmenopause , Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Alzheimer Disease, Huntington's Disease, Parkinson Disease und Epilepsie; von erythromelalgischem oder postpoliomyelitischem Schmerz, trigeminaler oder postherpetischer Neuralgie

Ein weiterer Gegenstand der Erfindung ist ein Verfahren zur Herstellung einer erfindungsgemäßen Thioaminosäure in einer Form, wie nachfolgend beschrieben.

Allgemeines Verfahren zur Darstellung der substituierten β-Thio-α-Aminosäuren

Für die synthetischen Arbeiten sind in der Literatur beschriebene Reaktionen angewandt, sowie im Hause bekannte Erfahrungen eingebracht worden.

**Schema1:**

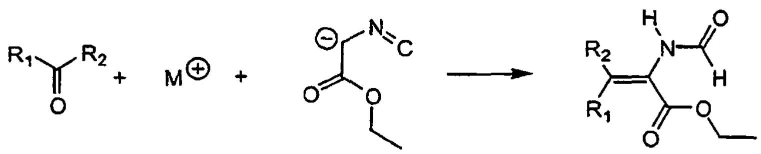

2 3

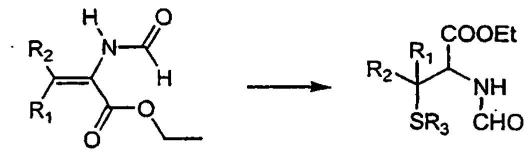

3 4

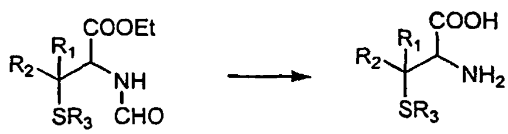

4 1

Die Deprotonierung des Isocyanessigsäurethylesters mit Basen wie Butyllithium, Natriumhydrid oder Kalium.-tert.butylat und anschließende Umsetzung mit Ketonen der allgemeinen Formel 2 in Tetrahydrofuran führt zu (E,Z)-2-Formylaminoacrylsäureethylestern der allgemeinen Formel 3. Durch Umsetzung von (E,Z)-2-Formylaminoacrylsäureethylestern der allgemeinen Formel 3 mit $P_4S_{10}$ in Toluol oder mit Mercaptanen der allgemeinen Formel $R_3SH$ in Gegenwart von Butyllithium in Toluol erhält man Formylamino-Ethylester der allgemeinen Formel 4. Reaktion der Formylamino-Ethylester der allgemeinen Formel 4 mit Salzäure führt zu den Thio-Aminosäuren der allgemeinen Formel 1. Die Diastereomerentrennung erfolgt auf geigneter Stufe mittels HPLC, Säulenchromatographie oder Kristallisation. Die Enantiomerentrennung erfolgt auf der Endstufe gleichfalls mittels HPLC, Säulenchromatographie oder Kristallisation. Man erhält nach diesem Verfahren die Aminosäuren der allgemeinen Formel 1 als Hydrochloride. Durch Basenfreisetzung oder Umfällung nach konventionellen Methoden erhält man weitere Salzformen.

Entsprechend ist Erfindungsgegenstand ein Verfahren zur Herstellung einer erfindungsgemäßen Thioaminsäure mit folgenden Schritten:

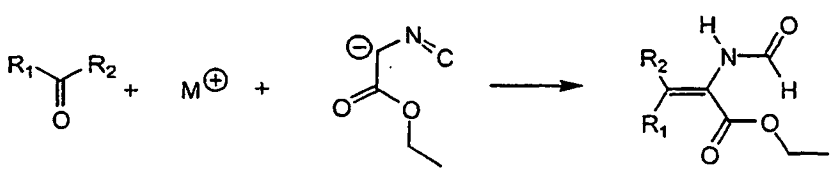

2 3

Deprotonierung des Isocyanessigsäurethylesters mit Basen, vorzugsweise Butyllithium, Natriumhydrid oder Kalium.-tert.butylat, und anschließende Umsetzung mit Ketonen der allgemeinen Formel 2 in Tetrahydrofuran führt zu

(E,Z)-2-Formylaminoacrylsäureethylestern der allgemeinen Formel 3,

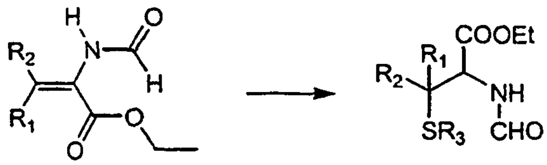

3 4

Umsetzung von (E,Z)-2-Formylaminoacrylsäureethylestern der allgemeinen Formel 3 mit $P_4S_{10}$ in Toluol oder mit Mercaptanen der allgemeinen Formel $R_3SH$ in Gegenwart von Butyllithium in Toluol, was zu Formylamino-Ethylester der allgemeinen Formel 4 führt,

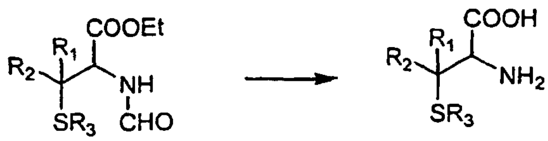

4 1

Reaktion der Formylamino-Ethylester der allgemeinen Formel 4 mit Säure, vorzugsweise Salzsäure, was zu den Thio-Aminosäuren der allgemeinen Formel1 bzw. I gemäß einem der Ansprüche 1 bis 4 führt, gegebenenfalls gefolgt oder unterbrochen von Diastereomerentrennung auf geigneter Stufe mittels HPLC, Säulenchromatographie oder Kristallisation bzw. gefolgt von Enantiomerentrennung mittels HPLC, Säulenchromatographie oder Kristallisation,
wobei R1 bis R3 die bereits oben genannte Bedeutung haben oder einem mit einer geigneten Schutzgruppe geschützten entsprechenden Rest entsprechen.

## Salzbildung

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo [*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure, in der sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon oder auch Wasser durchgeführt. Zur Herstellung der Hydrochloride eignet sich darüber hinaus Trimethylchlorsilan in wäßriger Lösung. Möglich ist auch die Überführung in basische Salze unter Verwendung von MetallIonen, z.B.: Alkali und Erdalkali-Ionen.
Im folgenden wird die Erfindung weiter durch Beispiele erläutert, ohne sie darauf zu beschränken.

## Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc. oder synthetisiert).
Die Analytik erfolgte über ESI-Massenspektrometrie oder HPLC.

**Synthesen:**

**Beispiel 1)**

**Synthetisierte Verbindungen:**

Repräsentative Beispiele erfindungsgemäßer Verbindungen sind folgende Verbindungen:

**Verbindung 1)**

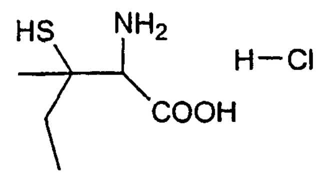

*rac*-2-Amino-3-mercapto-3-methyl-pentansäure Hydrochlorid als threo/erythro-Gemisch von 7:3

**Verbindung 2)**

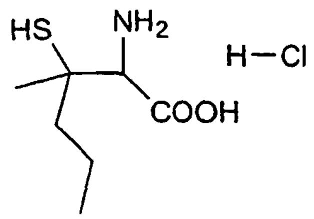

*rac*-2-Amino-3-mercapto-3-methyl-hexansäure Hydrochlorid als threo/erythro-Gemisch von 7:3

**Verbindung 3)**

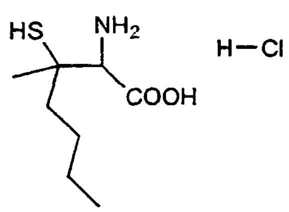

*rac*-2-Amino-3mercapto-3-methyl-heptansäure Hydrochlorid als threo/erythro:-Gemisch von 6:4

**Verbindung 4)**

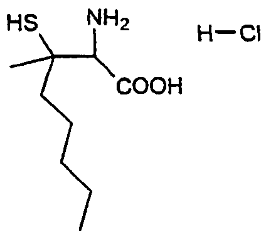

*rac*-2-Amino-3mercapto-3-methyl-octansäure Hydrochlorid als threo/erythro-Gemisch von 1:1

**Verbindung 5)**

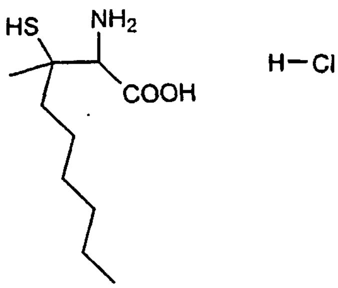

*rac*-2-Amino-3mercapto-3-methyl-nonansäure Hydrochlorid als threo/erythro-Gemisch von 6:4

**Verbindung 6)**

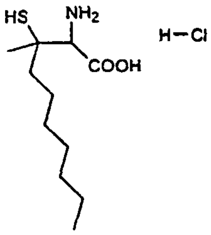

*rac*-2-Amino-3mercapto-3-methyl-decansäure Hydrochlorid als threo/erythro-Gemisch von 6:4

**Verbindung 7)**

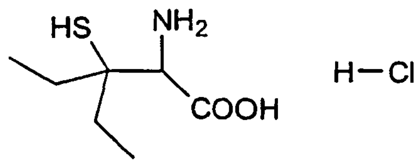

*rac*-2-Amino-3-ethyl-3-mercapto-pentansäure Hydrochlorid

**Verbindung 8)**

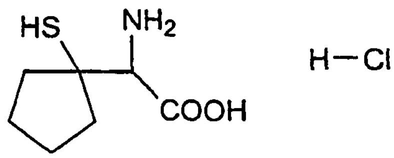

*rac*-Amino-(1-mercapto-cyclopentyl)essigsäure Hydrochlorid

**Verbindung 9)**

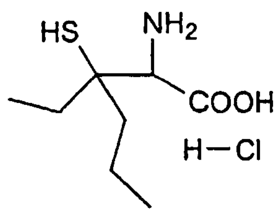

*rac*-Amino-3-ethyl-3-mercaptohexansäure Hydrochlorid als threo/erythro-Gemisch von 1:1

**Verbindung 10)**

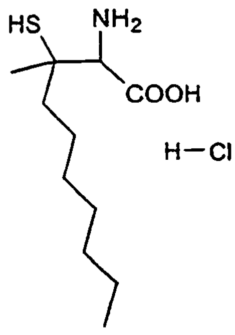

*rac-threo*-2-Amino-3-mercapto-3-methyl-decansäure Hydrochlorid

**Verbindung 11)**

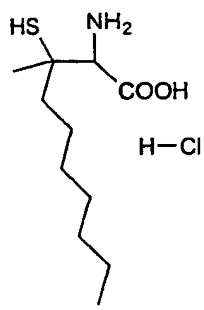

*rac*-erythro-2-Amino-3-mercapto-3-methyl-decansäure Hydrochlorid

**Verbindung 12)**

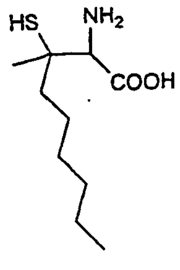

*rac-threo*-2-Amino-3-mercapto-3-methyl-nonansäure Hydrochlorid

**Verbindung 13)**

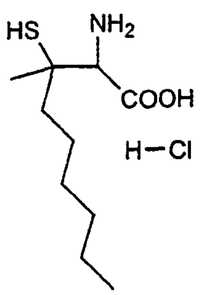

*rac-erythro*-2-Amino-3-mercapto-3-methyl-nonansäure Hydrochlorid

**Verbindung 14)**

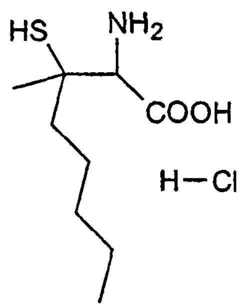

*rac*-threo-2-Amino-3-mercapto-3-methyl-octansäure Hydrochlorid

**Verbindung 15)**

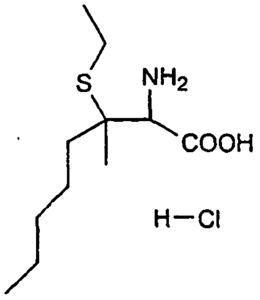

*rac*-2-Amino-3-ethylsulfanyl-3-methyl-octansäure Hydrochlorid als threo/erythro-Gemisch von 1:1

**Verbindung 16)**

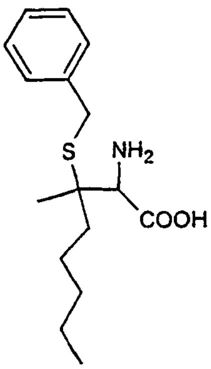

*rac-threo*-2-Amino-3-benzylsulfanyl-3-methyl-octansäure Hydrochlorid

**Verbindung 17)**

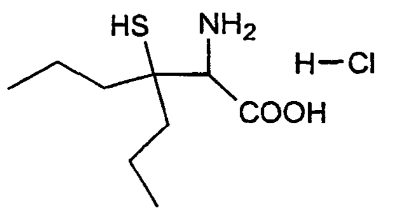

*rac*-2-Amino-3-mercapto-3-propyl-3-hexansäure Hydrochlorid

**Verbindung 18)**

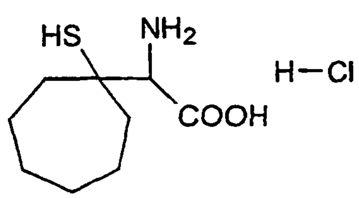

*rac*-Amino-(1-mercapto-cycloheptyl)-essigsäure Hydrochlorid

**Verbindung 19)**

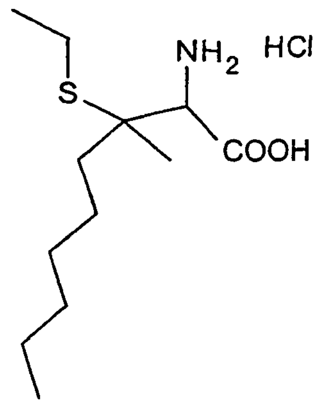

Verbindung 19

rac-2-Amino-3-ethylsulfanyl-3-methyl-nonansäure Hydrochlorid als threo/erythro-Gemisch von 6:4

**Verbindung 20)**

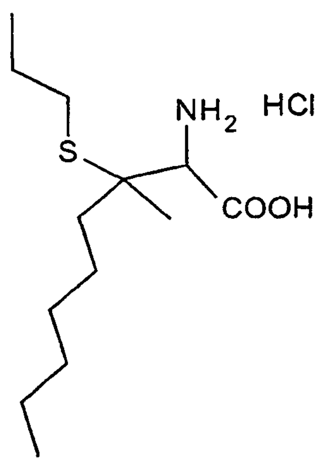

Verbindung 20

rac-2-Amino-3-methyl-3-propylsulfanyl-nonansäure Hydrochlorid als threo/erythro Gemisch von 6:4

**Verbindung 21)**

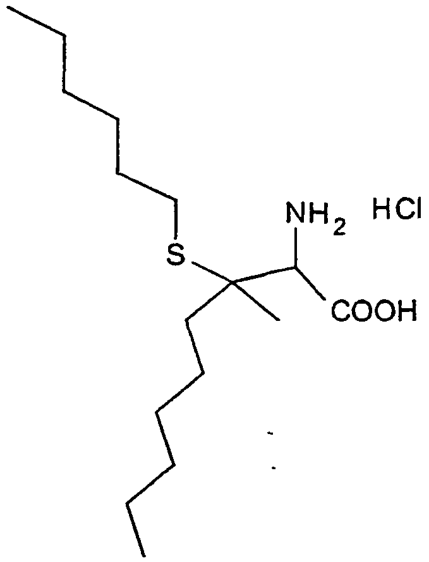

Verbindung 21

rac-2-Amino-3-hexylsulfanyl-3-methyl-nonansäure Hydrochlorid als threo/erythro-Gemisch von 6:4

**Verbindung 22)**

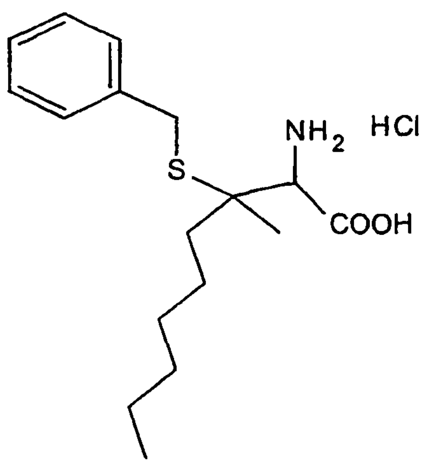

Verbindung 22

rac-2-Amino-3-benzylsulfanyl-3-methyl-nonansäure Hydrochlorid als threo/erythro-Gemisch von 6:4

**Verbindung 23)**

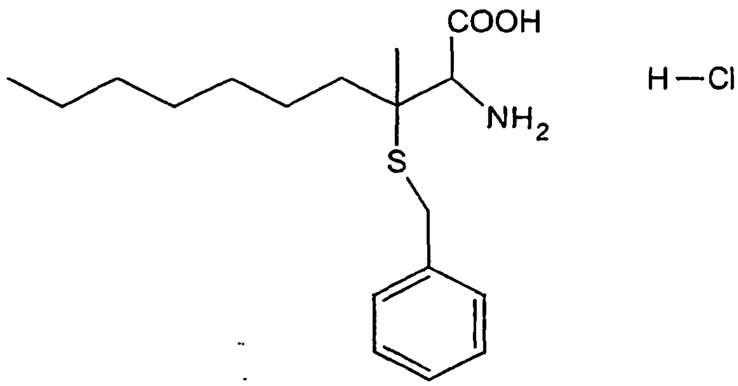

Verbindung 23

rac-2-Amino-3-benzylsulfanyl-3-methyl-decansäue Hydrochlorid als threo/erythro-Gemisch von 6:4

**Verbindung 24)**

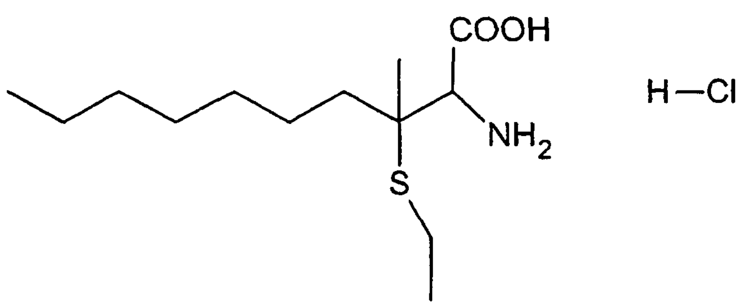

Verbindung 24

rac-2-Amino-3-ethylsulfanyl-3-methyl-decansäure Hydrochlorid als threo/erythro-Gemisch von 6: 4

**Verbindung 25)**

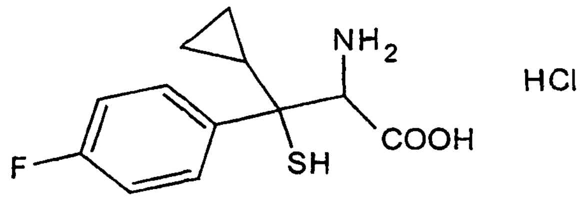

Verbindung 25

rac-2-Amino-3-cyclopropyl-3-(4-fluoro-phenyl)-3-mercapto-propansäre Hydrochlorid als threo/erythro-Gemisch von 6: 4

**Verbindung 26)**

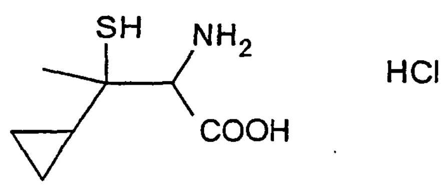

Verbindung 26

rac-2-Amino-3-cyclopropyl-3-mercapto-butansäure Hydrochlorid als threo/erythro-Gemisch von 6:4

**Verbindung 27)**

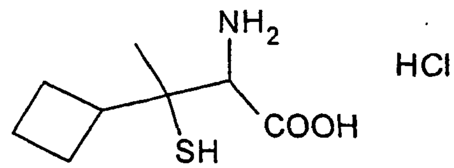

Verbindung 27

rac-2-Amino-3-cyclobutyl-3-mercapto-butansäure Hydrochlorid als threolerythro-Gemisch von 6:4

**Verbindung 28)**

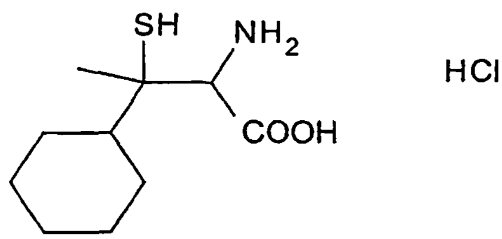

Verbindung 28

rac-2-Amino-3-cyclohexyl-3-mercapto-butansäure Hydrochlorid als threo/erythro-Gemisch von 6:4

**Verbindung 29)**

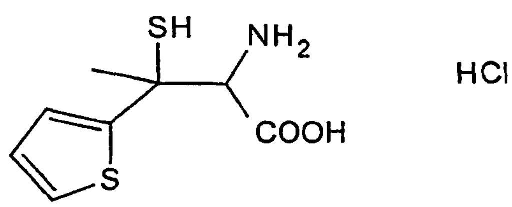

Verbindung 29

rac-2-Amino-3-mercapto-3-thiophen-2-yl-butansäure Hydrochlorid als threo/erythro-Gemisch von 6:4

**Verbindung 30)**

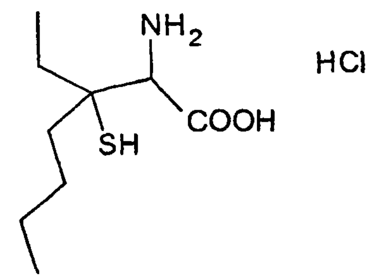

Verbindung 30

rac-2-Amino-3-ethyl-3-mercapto-heptansäure Hydrochlorid als threo/erythro-Gemisch von 6:4

**Verbindung 31)**

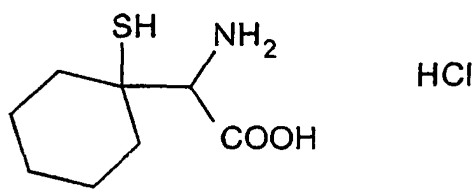

Verbindung 31

rac-Amino-(1-mercapto-cyclohexyl)- ethansäure Hydrochlorid

**Verbindung 32)**

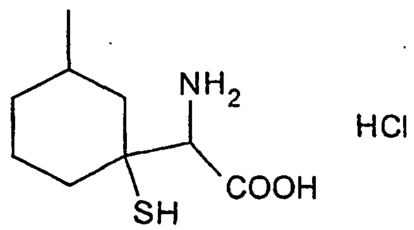

Verbindung 32

rac-Amino-(1-mercapto-3-methyl-cyclohexyl)-ethansäure Hydrochlorid

**Verbindung 33)**

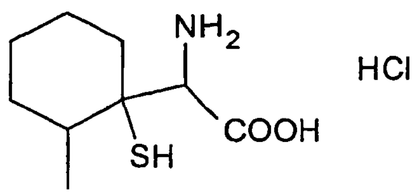

Verbindung 33

rac-Amino-(1-mercapto-2-methyl-cyclohexyl)-ethansäure Hydrochlorid

**Verbindung 34)**

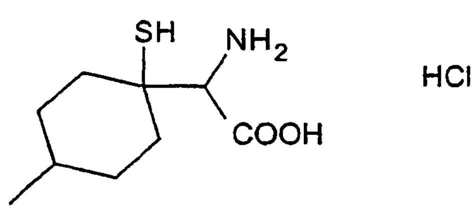

Verbindung 34

rac-Amino-(1-mercapto-4-methyl-cyclohexyl)-ethansäure Hydrochlorid

**Verbindung 35)**

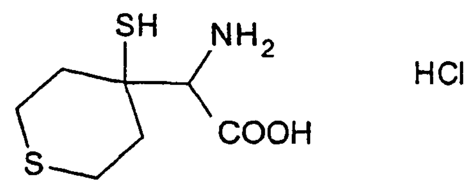

Verbindung 35

*rac*-Amino-(4-mercapto-tetrahydro-thiopyran-4-yl)-ethansäure Hydrochlorid

**Verbindung 36)**

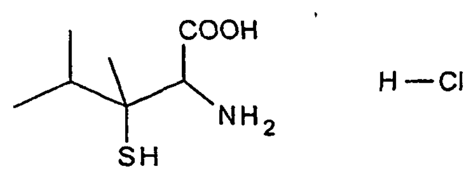

Verbindung 36

rac-2-Amino-3-mercapto-3,4-dimethyl-pentansäure Hydrochlorid als threo/erythro-Gemisch von 6:4

**Verbindung 37)**

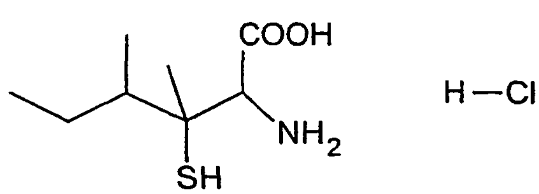

Verbindung 37

rac-2-Amino-3-mercapto-3,4-dimethyl-hexansäure Hydrochlorid als threo/erythro-Gemisch von 6:4

**Beispiel 2)**

**Herstellungsverfahren**

Die folgenden Beispiele dienen zur näheren Erläuterung des erfindungsgemäßen Verfahrens.

Die Ausbeuten der hergestellten Verbindungen sind nicht optimiert.

Alle Temperaturen sind unkorrigiert.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse - der Laufmittel für alle chromatographischen Untersuchungen sind stets in Volumen/Volumen angegeben.

Die Angabe Ether bedeutet Diethylether.

Soweit nicht anders angegeben, wurde Petrolether mit dem Siedebereich von 50°C-70°C benutzt.

**<u>Vorschrift 1</u>**

**Darstellung von Verbindung 6**

rac-2-Amino-3mercapto-3-methyl-decansäure Hydrochlorid als threo/erythro-Gemisch von 6:4; Verbindung 6 (Prod. 1)

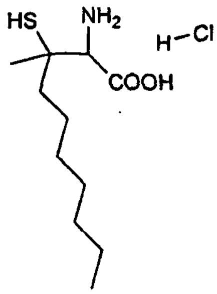

**1. Glycinethylester-Hydrochlorid (Prod. 2)**

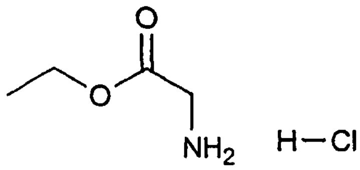

247.3 g Thionylchlorid und 130 g Glycin wurden bei -10 °C in 1000 ml Ethanol gegeben. Nach Entfernen des Eisbades wurde ein weiteres Äquivalent Glycin portionsweise hinzugegeben. Die Mischung wurde anschließend 2 h unter Rückfluß gerührt. Nach Abkühlen auf Raumtemperatur wurde am Rotationsverdampfer der überschüssige Alkohol und das Thionylchlorid entfernt. Der erhaltene weiße Feststoff wurde zweimal mit Ethanol versetzt und dieses wiederum am Rotationsverdampfer entfernt, um anhaftendes Thionylchlorid vollständig zu entfernen. Nach Umkristallisation aus Ethanol erhielt man 218.6 g (90.4% d.Th.) der Titelverbindung (Prod. 2).

**2. Formylaminoessigsäureethylester (Prod. 3)**

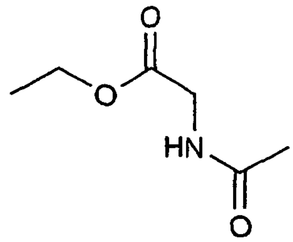

218 g Glycinethylester-Hydrochlorid (Prod. 2) wurden in 1340 ml Ethylformiat suspendiert. 223 mg Toluolsulfonsäure wurden zugeben und die Mischung wurde zum Rückfluß erhitzt. Nun wurden 178 g Triethylamin zu der siedenen Lösung zugetropft und die Reaktionslösung wurde über Nacht unter Rückfluß gerührt. Nach Abkühlen auf RT wurde das ausgefallene Ammoniumchlorid-Salz abfiltriert, das Filtrat wurde auf ca. 20% des Ursprungvolumens eingeengt und auf -5 °C gekühlt. Das erneut ausgefallene Ammoniumchlorid-Salz wurde abfiltriert, das Filtrat erneut eingeengt und bei 1 mbar destilliert. Man erhielt so 184 g (90.3% d. Th.) der Titelverbindung (Prod. 3).

**3. Isocyanessigsäureethylester (Prod. 4)**

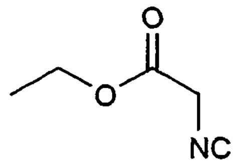

50 g Formylaminoessigsäureethylester (Prod. 3) und 104 g Diisopropylamin wurden in 400 ml Dichlormethan gegeben und auf -3 °C gekühlt. Dann wurden 70.1 g Phosphorylchlorid in 400 ml Dichlormethan hinzugetropft und anschließend wurde eine weitere Stunde bei dieser Temperatur gerührt. Nachdem das Eisbad entfernt und Raumtemperatur erreicht wurde, wurde vorsichtig mit 400 ml 20%iger Natriumcarbonat-Lösung hydrolysiert. Nach 60 minütigem Rühren bei RT wurden 400 ml Wasser und dann 200 ml Dichlormethan hinzugegeben. Die Phasen wurden getrennt und die organische Phase wurde zweimal mit je 100 ml 5%iger $Na_2CO_3$-Lösung gewaschen und über $MgSO_4$ getrocknet. Das Lösungsmittel wurde am Rotationsverdampfer evaporiert und das zurückbleibende braune Öl wurde destilliert. Man erhilet so 34.16 g (79.3% d. Th.) der Titelverbindung (Prod. 4).

**4. (*E*)und (*Z*)-2-Formylamino-3-methyldec-2-ensäure-ethylester (Prod. 5)**

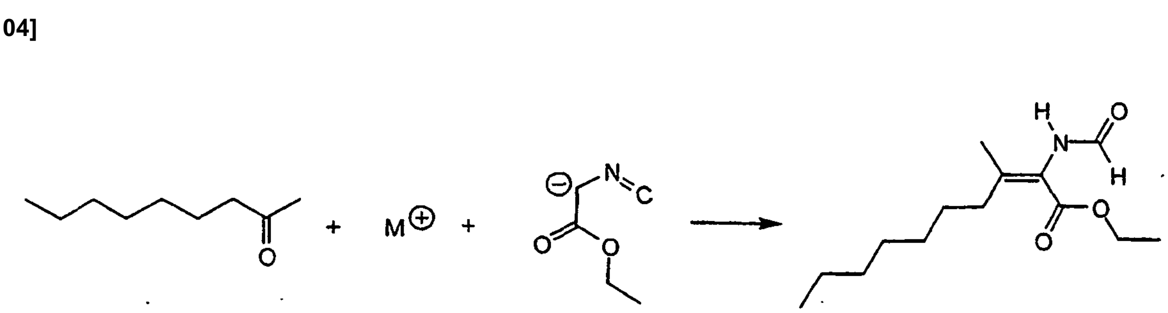

Zu einer Suspension von 23 g Kalium-tert.-butylat in 148 ml THF wurden bei -70°C bis -60°C unter Rühren eine Lösung von 22g Isocyanessigsäureethyl-ester (Prod. 4) in 49 ml THF eingetropft. Man ließ 20 min nachrühren;

anschließend wurden bei dieser Temperatur 27.7 g 2-Nonanon in 24 ml THF zugetropft. Nach Erwärmen auf RT wurden 11.7 ml Eisessig hinzugefügt. 15 min nach Zugabe des Eisessigs (DC-Kontrolle: Ether :Hexan 4:1) wurde das Lösemittel evaporiert. Der Rückstand wurde mit 300 ml Diethylether und 200 ml Wasser versetzt. Die organische Phase wurde abgetrennt und die wäßrige Phase wurde zweimal mit je 120 ml Ether gewaschen. Die vereinigten organischen Phasen wurden mit 80 ml 2N $NaHCO_3$-Lösung gewaschen und über $MgSO_4$ gerocknet. Anschließend wurde das Lösemittel evaporiert. Das so erhaltene Rohprodukt wurde mit 200 n-Hexan digeriert. Der Feststoff wurde abfiltriert viermal mit je 80 ml Hexan gewaschen und im Ölpumpenvakuum getrocknet. Man erhielt so 34.8 g (69.9% d.Th.) (*E*)- und (*Z*)-2-Formylamino-3-methyldec-2-ensäure-ethylester (Prod. 5) (E/Z-Verhälnis: 1:1) als weißen Feststoff.

**5. 2-Formylamino-3-mercapto-3-methyl-decansäureethylester als threo/erythro-Gemisch von 6:4 (Prod. 6)**

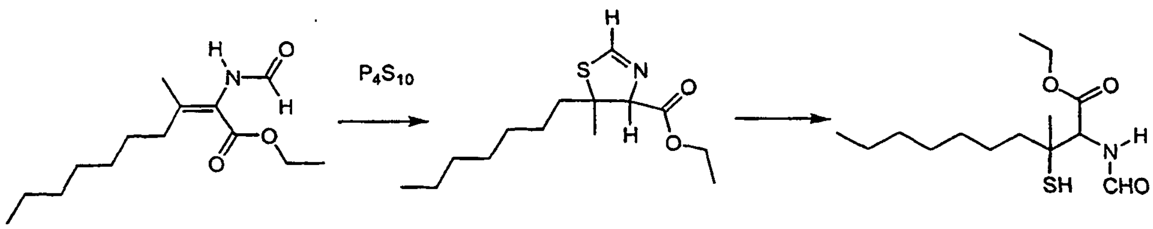


34.8 g (*E*)- und (*Z*)-2-Formylamino-3-methyldec-2-ensäure-ethylester (Prod. 5) (E/Z-Verhälnis: 1:1) wurden in 273 ml Toluol bei RT gelöst und anschließend mit 6.06 g $P_4S_{10}$ versetzt. Das Gemisch wurde unter Feuchtigkeitsausschluß 2 h bei 80°C gerührt (DC-Kontrolle: Essigester: Hexan 1:1). Anschließend wurde die entstandene Lösung auf RT abgekühlt und die organische Phase vom Lösemittel befreit. Das erhaltene Rohprodukt wurde in 300 ml Diethylether aufgenommen und mit 5 ml Wasser versetzt. Es wurde über Nacht nachgerührt. Das Wasser wurde abgetrennt und die organische Phase wurde über $MgSO_4$ getrockner und anschließend wurde das Lösemittel im Vakuum evaporiert. Man erhielt so 43 g 2-Formylamino-3-mercapto-3-methyl-decansäureethylester als threo/erythro-Gemisch von 6:4 (Prod. 6) als gelbes Öl. Dieses wurde an Kieselgel mit Diisopropylether, der 1 % 25%igen Ammoniak enthielt, chromatographiert. Man erhielt so 30 g (76% d.Th.) 2-Formylamino-3-mercapto-3-methyl-decansäureethylester als threo/erythro-Gemisch von 6:4 (Prod. 6) in Form eines farblosen Öls.

**6. *rac*-2-Amino-3mercapto-3-methyl-decansäure Hydrochlorid als threo/erythro-Gemisch von 6:4 (Prod. 1)**

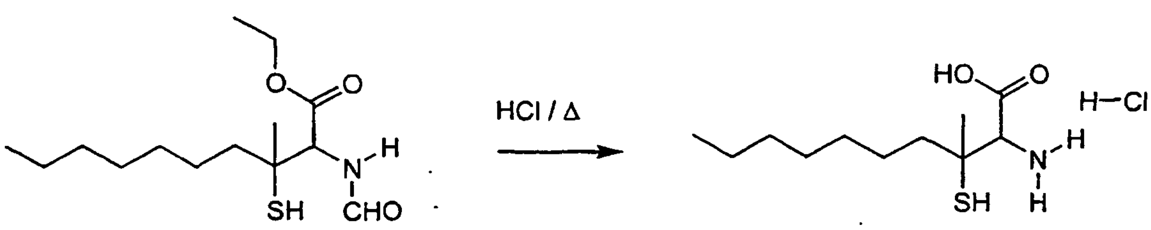


16.7 g 2-Formylamino-3-mercapto-3-methyl-decansäureethylester als threo/erythro-Gemisch von 6:4 (Prod. 6) wurden bei RT zu 606 ml 6N Salzsäure gegeben und anschließend 24 h unter Rückfluß gerührt (DC-Kontrolle: Dichlormethan : Methanol : Eisessig:35 : 5 : 3). Nach Abkühlen auf RT wurde unter Eiskühlung weitergerührt. Der ausgefallene weiße Feststoff wurde abgesaugt, mit Ether gewaschen und anschließend im Vakuum getrocknet. Man erhielt so 13.3 g (94.9% d.Th.)*rac*-2-Amino-3mercapto-3-methyl-decansäure Hydrochlorid als threo/erythro-Gemisch von 6:4 Verbindung 6 (Prod. 1).

**Vorschrift 2:**

**Darstellung von Verbindung 10 und Darstellung von Verbindung 11**

*rac-threo*-2-Amino-3-mercapto-3-methyl-decansäure Hydrochlorid Verbindung 10 (Prod. 7) und *rac-erythro*-2-Amino-3-mercapto-3-methyl-decansäure Hydrochlorid Verbindung 11 (Prod. 8).

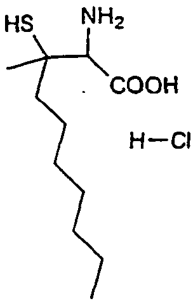

(Prod. 7)

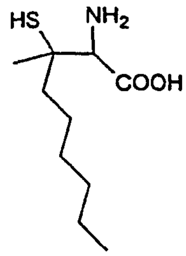

(Prod. 8)

Man erhielt *rac-threo*-2-Amino-3-mercapto-3-methyl-decansäure Hydrochlorid (Prod. 7) bzw. *erythro*-2-Amino-3-mercapto-3-methyl-decansäure Hydrochlorid (Prod. 8), indem man analog arbeitete wie in Vorschrift 1; Teil 1, 2, 3 und 4. Änderungen traten ab Teil 5 auf.

**5. *threo*-2-Formylamino-3-mercapto-3-methyl-decansäureethylester (Prod. 9) und *erythro*-2-Formylamino-3-mercapto-3-methyl-decansäureethylester (Prod. 10)**

34.8 g (E)- und (Z)-2-Formylamino-3-methyldec-2-ensäure-ethylester (Prod. 5) (E/Z-Verhälnis: 1:1) wurden in 273 ml Toluol bei RT gelöst und anschließend mit 6.06 g $P_4S_{10}$ versetzt. Das Gemisch wurde unter Feuchtigkeitsausschluß 2 h bei 80°C gerührt (DC-Kontrolle: Essigester: Hexan 1:1). Anschließend wurde die entstandene Lösung auf RT abgekühlt und die organische Phase vom Lösemittel befreit. Das erhaltene Rohprodukt wurde in 300 ml Diethylether aufgenommen und mit 5 ml Wasser versetzt. Es wurde über Nacht nachgerührt. Das Wasser wurde abgetrennt und die organische Phase wurde über $MgSO_4$ getrockner und anschließend wurde das Lösemittel im Vakuum evaporiert. Man erhielt so 43 g 2-Formylamino-3-mercapto-3-methyl-decansäureethylester als threo/erythro-Gemisch von 6:4 (6) als gelbes Öl. Dieses wurde an Kieselgel mit Diisopropylether, der 1 % 25%igen Ammoniak enthielt, chromatographiert. Man erhielt so 30 g (76% d.Th.) 2-Formylamino-3-mercapto-3-methyl-decansäureethylester als threo/erythro-Gemisch von 6:4 (Prod. 6) in Form eines farblosen Öls. Diese Mischfraktion wurde erneut an Kieselgel mit Diisopropylether, der 1 % 25%ige Ammoniaklösung enthielt, chromatographiert. Man erhielt so 5 g (12.7% d.Th.) *threo*-2-Formylamino-3-mercapto-3-methyl-decansäureethylester (Prod. 9) und 3.6 g (9.2% d.Th.) *erythro*-2-Formylamino-3-mercapto-3-methyl-decansäureethylester (Prod. 10).

**6. *rac-threo*-2-Amino-3-mercapto-3-methyl-decansäure Hydrochlorid (Prod. 7) und *rac-erythro*-2-Amino-3-mercapto-3-methyl-decansäure Hydrochlorid (Prod. 8)**

5 g *threo*-2-Formylamino-3-mercapto-3-methyl-decansäureethylester (**Prod**. 9) wurde bei RT zu 183 ml 6N Salzsäure gegeben bzw. 3.6 g *erythro*-2-Formylamino-3-mercapto-3-methyl-decansäureethylester (Prod. 10) wurde bei RT zu 132 ml 6N Salzsäure gegeben. Die weitere Vorgehensweise war identisch. Es wurde anschließend 24 h unter Rückfluß gerührt (DC-Kontrolle: Dichlormethan : Methanol : Eisessig:35 : 5 : 3). Nach Abkühlen auf RT wurde unter Eiskühlung weitergerührt. Der ausgefallene weiße Feststoff wurde abgesaugt, mit Ether gewaschen und anschließend im Vakuum getrocknet. Man erhielt so 4.2 g (94.9% d.Th.) *rac-threo*-2-Amino-3-mercapto-3-methyl-decansäure Hydrochlorid (Prod. 7) bzw. 3 g (94.9% d.Th.) *rac-erythro*-2-Amino-3-mercapto-3-methyl-decansäure Hydrochlorid (Prod. 8).

Vorschrift 3:

**Darstellung von Verbindung 1**

*rac*-2-Amino-3-mercapto-3-methyl-pentansäure Hydrochlorid als threo/erythro-Gemisch von 7:3; Verbindung 1 (Prod. 11)

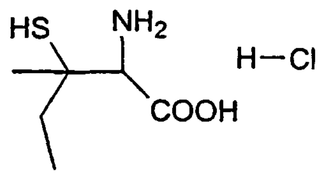

Durch Einsatz von 2-Butanon anstelle von 2-Nonanon in Vorschrift 1 erhielt man *rac*-2-Amino-3-mercapto-3-methyl-pentansäure Hydrochlorid als threo/erythro-Gemisch von 7:3; Verbindung 1 (Prod. 11).

Vorschrift 4:

**Darstellung von Verbindung 2:**

*rac*-2-Amino-3-mercapto-3-methyl-hexansäure Hydrochlorid als threo/erythro-Gemisch von 7:3; Verbindung 2 (Prod. 12)

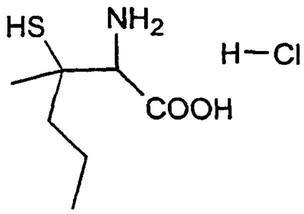

Durch Einsatz von 2-Pentanon anstelle von 2-Nonanon in Vorschrift 1 erhielt man *rac*-2-Amino-3-mercapto-3-methyl-hexansäure Hydrochlorid als threo/erythro-Gemisch von 7:3 (Prod. 12)

Vorschrift 5:

**Darstellung von Verbindung 3:**

*rac*-2-Amino-3mercapto-3-methyl-heptansäure Hydrochlorid als threo/erythro:-Gemisch von 6:4; Verbindung 3 (Prod. 13)

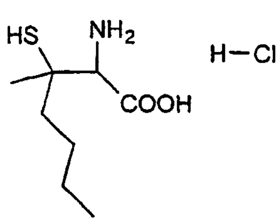

Durch Einsatz von 2-Hexanon anstelle von 2-Nonanon in Vorschrift 1 erhielt man *rac*-2-Amino-3-mercapto-3-methyl-heptansäure Hydrochlorid als threo/erythro:-Gemisch von 6:4; Verbindung 3 (Prod. 13)

**Vorschrift 6:**

**Darstellung von Verbindung 4:**

*rac*-2-Amino-3mercapto-3-methyl-octansäure Hydrochlorid als threo/erythro-Gemisch von 1:1; Verbindung 4 (Prod. 14)

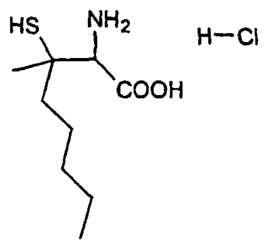

Durch Einsatz von 2-Heptanon anstelle von 2-Nonanon in Vorschrift 1 erhielt man *rac*-2-Amino-3mercapto-3-methyl-octansäure Hydrochlorid als threo/erythro-Gemisch von 1:1; Verbindung 4 (Prod. 14)

**Vorschrift7:**

**Darstellung von Verbindung 14**

*rac-threo*-2-Amino-3mercapto-3-methyl-octansäure Hydrochlorid; Verbindung (Prod. 15)

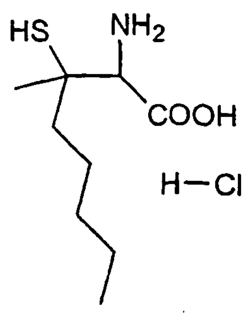

Durch Einsatz von 2-Heptanon anstelle von 2-Nonanon in Vorschrift 2 erhielt man *rac-threo*-2-Amino-3mercapto-3-methyl-octansäure Hydrochlorid; Verbindung (Prod.15)

**Vorschrift 8:**

**Darstellung von Verbindung 5**

*rac*-2-Amino-3mercapto-3-methyl-nonansäure Hydrochlorid als threo/erythro-Gemisch von 6:4; Verbindung 5 (Prod. 16)

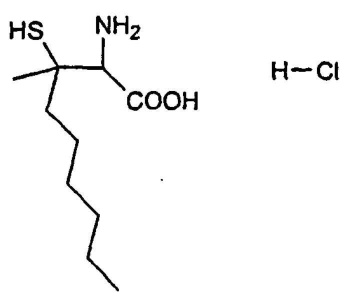

Durch Einsatz von 2-Octanon anstelle von 2-Nonanon in Vorschrift 1 erhielt man *rac*-2-Amino-3mercapto-3-methyl-nonansäure Hydrochlorid als threo/erythro-Gemisch von 6:4; Verbindung 5 (Prod.16).

__Vorschrift 9:__

**Darstellung von Verbindung 12 und Verbindung 13**

*rac*-threo-2-Amino-3-mercapto-3-methyl-nonansäure Hydrochlorid; Verbindung 12 (Prod. 17) und *rac*-erythro-2-Amino-3-mercapto-3-methyl-nonansäure Hydrochlorid; Verbindung 13 (Prod. 18)

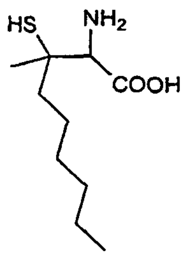

(12)

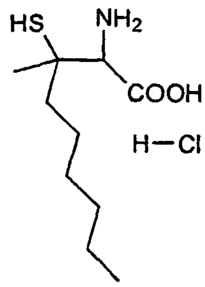

(13)

Durch Einsatz von 2-Octanon anstelle von 2-Nonanon in Vorschrift 2 erhielt man *rac-threo*-2-Amino-3-mercapto-3-methyl-nonansäure Hydrochlorid; Verbindung 12 (Prod. 17) und *rac-erythro*-2-Amino-3-mercapto-3-methyl-nonansäure Hydrochlorid; Verbindung 13 (Prod. 18).

__Vorschrift 10:__

**Darstellung von Verbindung 7**

*rac*-2-Amino-3-ethyl-3-mercapto-pentansäure Hydrochlorid; Verbindung 7 (Prod. 19)

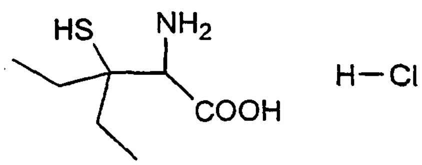

Durch Einsatz von 3-Pentanon anstelle von 2-Nonanon in Vorschrift 1 erhielt man *rac*-2-Amino-3-ethyl-3-mercapto-pentansäure Hydrochlorid; Verbindung 7 (Prod. 19).

__Vorschrift 11:__

**Darstellung von Verbindung 8**

*rac*-Amino-(1-mercapto-cyclopentyl)essigsäure Hydrochlorid; Verbindung 8 (Prod. 20)

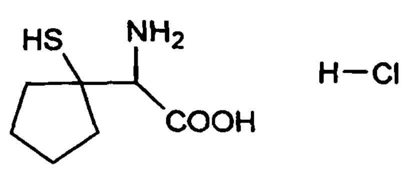

Durch Einsatz von Cyclopentanon von 2-Nonanon in Vorschrift 1 erhielt man *rac*-Amino-(1-mercapto-cyclopentyl)essigsäure Hydrochlorid; Verbindung 8 (Prod. 20).

**Vorschrift 12:**

**Darstellung von Verbindung 9**

*rac*-Amino-3-ethyl-3-mercaptohexansäure Hydrochlorid als threo/erythro-Gemisch von 1:1; Verbindung 9 (Prod. 21)

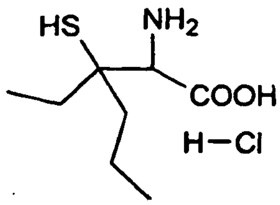

Durch Einsatz von 3-Hexanon anstelle von 2-Nonanon in Vorschrift 1 erhielt man *rac*-Amino-3-ethyl-3-mercaptohexansäure Hydrochlorid als threo/erythro-Gemisch von 1:1; Verbindung 7 (Prod. 21).

**Vorschrift 13:**

**Darstellung von Beispiel 17**

*rac*-2-Amino-3-mercapto-3-propyl-3-hexansäure Hydrochlorid (22)

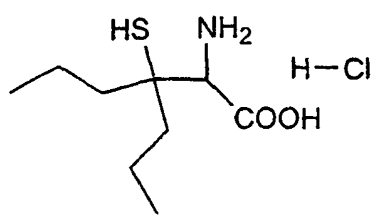

Durch Einsatz von 4-Heptanon anstelle von 2-Nonanon in Vorschrift 1 erhielt man *rac*-2-Amino-3-mercapto-3-propyl-3-hexansäure Hydrochlorid (22).

**Vorschrift 14:**

**Darstellung von Verbindung 18**

*rac*-Amino-(1-mercapto-cycloheptyl)-essigsäure Hydrochlorid; Verbindung 18 (Prod. 23)

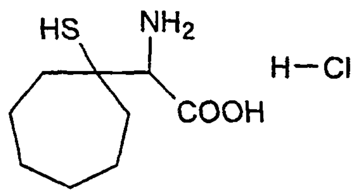

Durch Einsatz von Cycloheptanon anstelle von 2-Nonanon in Vorschrift 1 erhielt man *rac*-Amino-(1-mercapto-cycloheptyl)-essigsäure Hydrochlorid; Verbindung 7 (Prod. 23).

**Vorschrift 15:**

**Darstellung von Verbindung 15**

*rac*-2-Amino-3-ethylsulfanyl-3-methyl-octansäure Hydrochlorid als threo/erythro-Gemisch von 1:1; Verbindung 15 (Prod. 24)

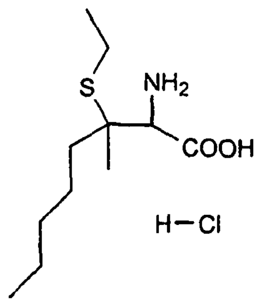

Die Vorgehensweise ist identisch zu der in Vorschrift 1; Teil1; 2 und 3. Ab Teil 4 ergeben sich Unterschiede.

**4. (*E*)und (*Z*)-2-Formylamino-3-methyl-oct-2-ensäure-ethylester (Prod. 25)**

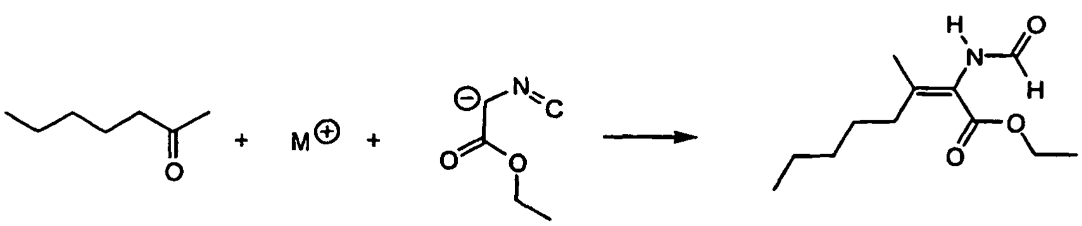

Zu einer S,uspension von 23 g Kalium-tert.-butylat in 148 ml THF wurden bei -70°C bis -60°C unter Rühren eine Lösung von 22g Isocyanessigsäureethyl-ester (Prod. 4) in 49 ml THF eingetropft. Man ließ 20 min nachrühren; anschließend wurden bei dieser Temperatur 27.7 g 2-Heptanon in 24 ml THF zugetropft. Nach Erwärmen auf RT wurden 11.7 ml Eisessig hinzugefügt. 15 min nach Zugabe des Eisessigs (DC-Kontrolle: Ether :Hexan 4:1) wurde das Lösemittel evaporiert. Der Rückstand wurde mit 300 ml Diethylether und 200 ml Wasser versetzt. Die organische Phase wurde abgetrennt und die wäßrige Phase wurde zweimal mit je 120 ml Ether gewaschen. Die vereinigten organischen Phasen wurden mit 80 ml 2N $NaHCO_3$-Lösun gewaschen und über $MgSO_4$ gerocknet. Anschließend wurde das Lösemittel evaporiert. Das so erhaltene Rohprodukt wurde mit 200 n-Hexan digeriert. Der Feststoff wurde abfiltriert viermal mit je 80 ml Hexan gewasche und im Ölpumpenvakuum getrocknet. Man erhielt so 34.8 g (69.9% d.Th.) (E)- und (Z)-2-Formylamino-3-methyl-oct-2-ensäure-ethylester (Prod. 25) (E/Z-Verhälnis: 1:1) als weißen Feststoff.

**5. 3-Ethylsulfanyl-2-formylamino-3-methyl-octansäure-ethylester als threo/erythro-Gemisch von 1:1 (Prod. 26)**

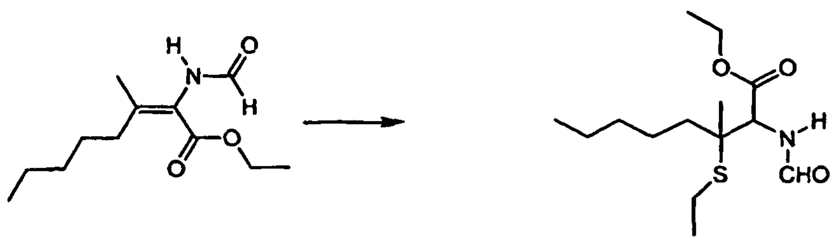

0.28 ml Butyllithium wurden in 40 ml absolutem THF gegeben und die Mischung wurde auf 0 °C gekühlt. Nun wurden 2.73 g Ethylmercaptan hinzugetropft. Nach 20 minütigem Rühren wurde die Lösung auf eine Temperatur zwischen -40 und 0 °C gekühlt und es wurde eine Lösung von 1 g (*E*)und(*Z*)-2-Formylamino-3-methyloct-2-ensäure-ethylester (E/Z-Verhältnis: 1:1) (Prod. 25) langsam hinzugegeben. Es wurde 2 h bei der Temperatur gerührt und danach auf 0 °C erwärmt und anschließend wurde mit 100 ml einer 5%iger Natriumhydroxid-Lösung hydrolysiert. Die Phasen wurden getrennt und die wäßrige Phase wurde zweimal mit je 100 ml Dichlormethan extrahiert. Die vereinigten organischen Phasen wurden über $MgSO_4$ getrocknet und das Lösungsmittel am Rotationsverdampfer entfernt. Das im Überschuß eingesetzte Mercaptan konnte mittels Chromatographie an Kieselgel mit Dichlormethan/Diethylether (6:1) als Eluent abgetrennt werden. Man erhielt so die Titelverbindung (Prod. 26) als farbloses Öl in einer Ausbeute von 1.05 g (82% d.Th.).

**6. *rac*-2-Amino-3-ethylsulfanyl-3-methyl-octansäure Hydrochlorid als threo/erythro-Gemisch von 1:1 (Prod. 24)**

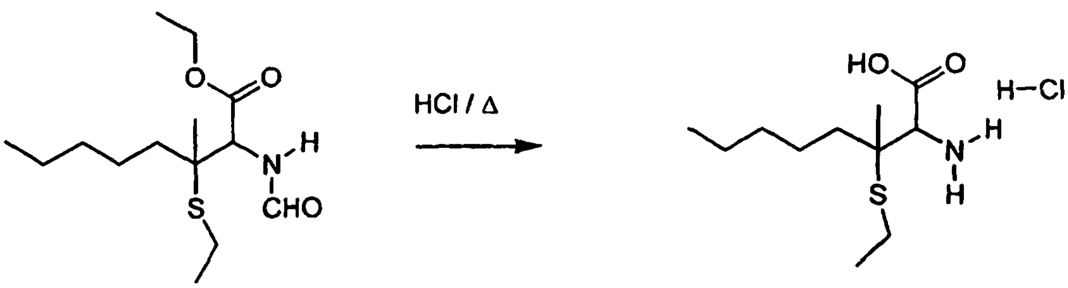


1.05 g 3-Ethylsulfanyl-2-formylamino-3-Methyl-octansäure-ethylester als threo/erythro-Gemisch von 1:1 (Prod. 26) wurden bei RT zu 40 ml 6N Salzsäure gegeben und anschließend 24 h unter Rückfluß gerührt (DC-Kontrolle: Dichlormethan : Methanol : Eisessig:35 : 5 : 3). Nach Abkühlen auf RT wurde unter Eiskühlung weitergerührt. Der ausgefallene weiße Feststoff wurde abgesaugt, mit Ether gewaschen und anschließend im Vakuum getrocknet. Man erhielt so 0.8 g (94.9% d.Th.) *rac*-2-Amino-3-ethylsulfanyl-3-methyl-octansäure Hydrochlorid als threo/erythro-Gemisch von 1:1; Verbindung 15 (Prod. 24).

__Vorschrift 16:__

**Darstellung von Verbindung 16**

*rac-threo*-2-Amino-benzylsulfanyl-methyl-octansäure Hydrochlorid; Verbindung 16 (Prod. 27)

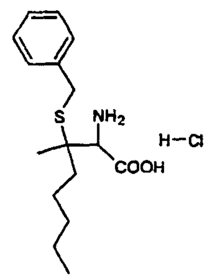

Die Vorgehensweise ist identisch mit der in Vorschrift 15; Teil 1, 2, 3 und 4. Unterschiede treten ab Teil 5 auf.

**5. threo-3-Benzylsulfanyl-2-formylamino-3-methyl-octansäure-ethylester (Prod. 28)**

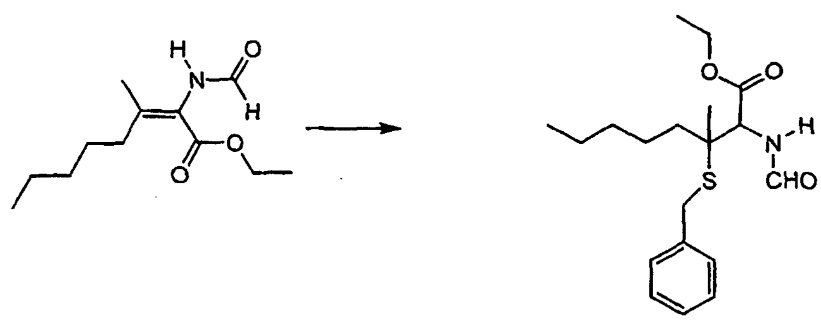

0.28 ml n-Butyllithium wurden in 40 ml absolutem THF gegeben und die Mischung wurde auf 0 °C gekühlt. Nun wurden 5.5 g Benzylmercaptan hinzugetropft. Nach 20 minütigem Rühren wurde die Lösung auf eine Temperatur zwischen -40 und 0 °C gekühlt und es wurde eine Lösung von 1 g (*E*)und(*Z*)-2-Formylamino-3-methyloct-2-ensäure-ethylester (E/Z-Verhältnis: 1:1) langsam hinzugegeben. Es wurde 2 h bei der Temperatur gerührt und danach auf 0 °C erwärmt und anschließend wurde mit 100 ml einer 5%iger Natriumhydroxid-Lösung hydrolysiert. Die Phasen wurden getrennt und die wäßrige Phase wurde zweimal mit je 100 ml Dichlormethan extrahiert. Die vereinigten organischen Phasen wurden über $MgSO_4$ getrocknet und das Lösungsmittel am Rotationsverdampfer entfernt. Das im Überschuß eingesetzte Mercaptan konnte mittels Chromatographie an Kieselgel mit Dichlormethan/Diethylether (6:1) als Eluent abgetrennt werden. Durch Kristallisation aus Pentan/Ethanol (10:1) erhielt man die Titelverbindung (Prod. 28) als weißen Feststoff in einer Ausbeute von 1.51 g (98% d.Th.).

**6. *rac-threo*-2-Amino-benzylsulfanyl-methyl-octansäure Hydrochlorid (Prod. 27)**

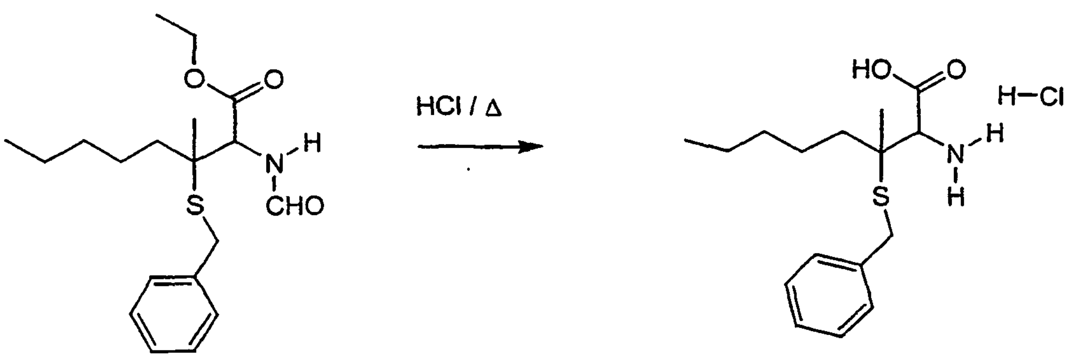


1.51 g threo-3-Benzylsulfanyl-2-formylamino-3-methyl-octansäure-ethylester (Prod. 28) wurden bei RT zu 40 ml 6N Salzsäure gegeben und anschließend 24 h unter Rückfluß gerührt (DC-Kontrolle: Dichlormethan : Methanol : Eisessig:35 : 5 : 3). Nach Abkühlen auf RT wurde unter Eiskühlung weitergerührt. Der ausgefallene weiße Feststoff wurde abgesaugt, mit Ether gewaschen und anschließend im Vakuum getrocknet. Man erhielt so 0.9 g (94.9% d.Th.) *rac-threo*-2-Amino-benzylsulfanylmethyl-octansäure Hydrochlorid; Verbindung 16 (Prod. 27).

**Pharmakologische Untersuchungen**

**Beispiel 3:**

**Bindungsassay**

Beim Bindungsassay wird Gabapentin benutzt, um die Bindung und Affinitäten der ausgewählten Verbindungen zu überprüfen. Die Affinität der erfindungsgemäßen Verbindungen wird über die Verdrängung von Gabapentin von seiner Bindungsstelle gemessen. Wenn die ausgewählten Verbindungen Gabapentin von seiner Bindungsstelle verdrängen können, so kann man erwarten, daß sie dem Gabapentin vergleichbare pharmakologische Eigenschaften entfalten z.B. als Agenz gegen Schmerz oder Epilepsie. Die erfindungsgemäßen Verbindungen zeigen eine gute HemmungNerdrängung von Gabapentin in diesem Assay. Die untersuchten Verbindungen weisen daher in diesem biochemischen Assay eine Affinität zur bislang unbekannten Gabapentin-Bindungsstelle auf. Die Affinitäten beziehungsweise die Prozent Hemmung der Verbindungen in Bezug auf die Gabapentinbindung sind der Tabelle 1 zu entnehmen:

Tabelle 1:

| Verbindung Nr. | Affinität (IC$_{50}$) nM und/oder %-Hemmung (Konz.) |
|---|---|
| 1 | 268 |
| 2 | 165 |
| 3 | 280 oder 99.7% ($10^{-5}$µM) |
| 4 | 186 |
| 5 | 70 |
| 6 | 199 |
| 7 | 258 |
| 8 | 151 |
| 9 | 339 oder 97.5% (10-5 µM) |
| 10 | 150 |

| | |
|---|---|
| 11 | 120 |
| 12 | 70 |
| 13 | 30 |
| 14 | 100 |
| 15 | 92% ($10^{-5}$µM) |
| 16 | 1800 oder 93% ($10^{-5}$ µM) |
| 17 | 2350 |
| 18 | 15% ($10^{-5}$ µM) |
| 19 | 271 |
| 20 | 3050 |
| 21 | 12400 |
| 22 | 336 |
| 23 | 91% ($10^{-5}$ µM) |
| 24 | 90% ($10^{-5}$ µM) |
| 25 | 40% ($10^{-5}$ µM) |
| 26 | 703 |
| 27 | 589 |
| 28 | 1320 |
| 29 | 30% ($10^{-5}$ µM) |
| 30 | 314 |
| 31 | 187 |
| 32 | 223 |
| 33 | 528 |
| 34 | 1004 |
| 35 | 84% ($10^{-5}$ µM) |
| 36 | 88% ($10^{-5}$ µM) |
| 37 | 196 |

**Beispiel 4:**

**Analgesieprüfung im Writhing-Test an der Maus**

Die antinociceptive Wirksamkeit der erfindungsgemäßen Verbindungen wurde im Phenylchinon-induzierten Writhing-Test, modifiziert nach I.C. Hendershot, J. Forsaith, J.Pharmacol. Exp. Ther. 125, 237 - 240 (1959) an der Maus untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0.3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die physiologische Kochsalzlösung i.v. und Phenyylchinon i.v. erhielten.

Alle Substanzen wurden in der Standarddosis von 10 mg/kg getestet. Die prozentuale Hemmung (% Hemmung) der Writhingreaktionen durch eine Substanz wurde nach folgender Formel berechnet:

%Hemmung = 100 -[WR behandelte Tiere/WR Kontrolle X 100]

Alle untersuchten erfindungsgemäßen Verbindungen zeigten eine Wirkung im Writhing Test.

Die Ergebnisse ausgewählter Writhing-Untersuchungen sind in der Tabelle 2 zusammengefaßt. Gabapentin zeigt einen $ED_{50}$ von 38 mg/kg.

Tabelle 2: Analgesieprüfung im Writhing-Test an der Maus

| Verbindung Nr. | Writhing Maus i.v. $ED_{50}$ |
|---|---|
| 4 | 12 mg/kg |
| 6 | 35 mg/kg |
| 8 | 70 mg/kg |

**Beispiel 5:**

**Formalin Test Maus**

Die Untersuchungen zur Bestimmung der antinociceptiven Wirkung der erfindungsgemäßen Verbindungen wurden im Formalin-Test an männlichen Albino-Mäusen (NMRI, 25 - 35 g, Iffa Credo, Belgien) durchgeführt.

Im Formalin-Test werden die erste (frühe) Phase (0-15 min nach Formalin-Injektion) und die zweite (späte) Phase (15-60 min nach Formalin-Injektion) unterschieden (D. Dubuisson er al, Pain, Vol. 4, pp. 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre et al, Pain, Vol. 52, pp. 259 - 285 (1993)).

Die erfindungsgemäßen Verbindungen wurden in der zweiten Phase des Formalin-Tests untersucht, um Aussagen über Substanzwirkungen im chronisch/entzündlichen Schmerz zu erhalten.

Durch eine einmalige subkutane Formalin-Injektion (20 µl, 1 %ige wäßrige Lösung) in die dorsale Seite der rechten Hinterpfote wurde bei freibeweglichen Versuchstieren eine nociceptive Reaktion induziert, die sich in deutlichem Lekken und Beißen der betroffenen Pfote äußert.

Für den Untersuchungszeitraum in der zweiten (späten) Phase des Formalin-Tests wurde das nozizeptive Verhalten durch Beobachtung der Tiere kontinuierlich erfaßt. Die Quantifizierung des Schmerzverhaltens erfolgte durch Summation der Sekunden, in denen die Tiere im Untersuchungszeitraum Lecken und Beißen der betroffenen Pfote zeigten. Nach Injektion von Substanzen, die im Formalin-Test antinozizeptiv wirksam sind, sind die beschriebenen Verhaltensweisen der Tiere reduziert, evtl. sogar aufgehoben. Entsprechend den Substanzversuchen, bei denen die Tiere Testsubstanz vor Formalin injiziert bekommen hatten, wurde den Kontrolltieren Vehikel, d.h. Lösungmittel (z.B. 0,9%ige NaCl-Lösung), vor der Formalin-applikation gespritzt. Das Verhalten der Tiere nach Substanzgabe (n=10 pro Substanzdosierung) wurde mit einer Kontrollgruppe (n=10 verglichen).

Basierend auf der Quantifizierung des Schmerzverhaltens wurde die Substanzwirkung im Formalin-Test als Änderung der Kontrolle in Prozent ermittelt. Die $ED_{50}$-Berechnungen erfolgten mittels Regressionsanalyse. Abhängig

von der Applikationsart der erfindungsgemäßen Verbindungen wurde der Applikationszeitpunkt vor der Formalin-Injektion gewählt (intraperitoneal: 15 min, intravenös: 5 min).

Die erfindungsgemäßen Verbindungen zeigten eine Hemmung der durch Formalin induzierten Nociception. Die entsprechenden Ergebnisse im Formalin-Test an der Maus sind in der nachfolgenden Tabelle 3 zusammengefaßt. Gabapentin zeigt einen ED50 von 79 mg/kg

Tabelle 3: Analgesieprüfung im Formalin Test Maus

| Verbindung Nr. | Formalin Test Maus $ED_{50}$ |
|---|---|
| 2 | 158 mg/kg (i.v.) |
| 4 | 67 mg/kg (i.v.) |
| 5 | 54 mg/kg (i.p.) |
| 6 | 66 mg/kg (i.v.) |
| 8 | 79 mg/kg (i.v.) |
| 10 | 105 mg/kg i.p. |
| 12 | 78 mg/kg i.p. |

**Beispiel 6**

**Bennett / Neuropathischer Schmerz an der Ratte**

Die Untersuchung auf Wirksamkeit im neuropathischen Schmerz wurde im Bennett-Modell (chronic constriction injury; Bennett und Xie, 1988, Pain 33: 87-107) untersucht.

Sprague-Dawley Ratten mit einem Gewicht von 140-160g werden unter Nembutal-Narkose mit vier losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa vier Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen Zeitraum von einer Stunde an vier Zeitpunkten (15, 30, 45, 60 min. nach Applikation) bestimmt und die resultierend Fläche unter der Kurve (AUD) sowie die Hemmung der Kälte-Allodynie zu den einzelnen Meßpunkten in Prozent Wirkung zur Vehikelkontrolle (AUD) bzw. zum Ausgangswert (Einzelmeßpunkte) ausgedrückt. Die Gruppengröße beträgt n=10, die Signifikanz einer anti-allodynischen Wirkung wird anhand der AUD-Werte über einen gepaarten T-Test (* $0.05 \geq p > 0.01$; ** $0.01 \geq p > 0.001$; *** $p \leq 0.001$; Armitage und Berry, 1987, Stat. Methods in Medical Research, London: Blackwell Scientific Publications) bestimmt.

Die untersuchte erfindungsgemäße Verbindungen zeigte eine anti-allodynische Wirkung. Die Ergebnisse sind im Vergleich zu Gabapentin in der nachfolgenden Tabelle 4 zusammengefaßt.

Tabelle 4: Prüfung der Hemmung im neuropathischen Schmerz an der Ratte

| Verbindung | Dosis [mg/kg] i.p. | AUD | Änderung gegen Kontolle(%) |
|---|---|---|---|
| Gabapentin | 100 | 1940.3 +/-139.7*** | 34.5 |
| Gabapentin | 464 | 2577.8 +/-147.4*** | 47.3 |
| Verbindung 4 | 46.4 | 1893.1 +/- 284.6*** | 32.5 |
| Verbindung 4 | 100 | 3603.1 +/- 228.1*** | 66.9 |

**Beispiel 7**

**Mechanische Hyperalgesie nach Pfoteninzision an der Ratte (Paw incision-Modell):**

**1. EINLEITUNG**

In diesem Modell wird der Wundschmerz in der Umgebung einer Inzision an der plantaren Seite einer Hinterpfote der Ratte als Modell für postoperativen Schmerz untersucht (Brennan, T.J., Vandermeulen, E.P., Gebhart, G. F., Pain (1996) 493-501). Für diese Zwecke wird die Wegziehlatenz nach punktförmiger mechanischer Stimulation mit einem elektronischen von Frey-Filament bestimmt. Nach der Pfoteninzision entwickelt sich eine mechanische Hyperalgesie, die über mehrere Tage stabil bliebt.

**2. Material und Methoden**

**Pfoteninzision:**

Es werden männliche Sprague Dawley Ratten (Körpergewicht 200-300 g) verwendet. Unter Halothannarkose wird eine 1 cm lange Inzision, beginnend 0.5 cm von dem proximalen Ende der Ferse, durch Haut, Faszie und M. plantaris gesetzt und mit zwei Nähten verschlossen.

**3. Versuchsdurchführung:**

Unter Verwendung eines elektronischen von Frey-Filamentes (Digital Transducer Indicator Model 1601C, IITC inc.) wird die Wegziehschwelle der Pfote, ausgedrückt in Gramm, nach punktförmiger mechanischer Stimulation bestimmt. Dazu wird die Wegziehschwelle pro Meßpunkt fünfmal im Abstand von 30 sec gemessen und der individuelle Median bestimmt, anhand derer wiederum der Mittelwert des Tierkollektivs berechnet wird. Pro Versuchstiergruppe werden 10 Ratten getestet.

Zur Untersuchung der *Primären Hyperalgesie* wird die Wegziehschwelle an der ipsilateralen Pfote in unmittelbarer Nähe zur Inzision sowie in derselben Position an der kontralateralen Pfote bestimmt. Die Messungen erfolgen zweimal vor dem operativen Eingriff zur Bestimmung des Vortestmittelwertes, postoperativ unmittelbar vor der Substanzgabe sowie zu verschiedenen Zeitpunkten nach Substanzgabe (i.d.R. 15, 30, 60, 90, 120 min p.appl.). Die Untersuchungen können von Substanzen in einem Zeitraum von 2 Stunden bis zu 3 Tagen postoperativ erfolgen.

**4. Auswertung:**

DIE WIRKSAMKEIT EINER SUBSTANZ WIRD ANHAND DER BEEINFLUSSUNG DER WEGZIEHSCHWELLE DER IPSILATERALEN PFOTE BESCHRIEBEN:

$$\%MPE = 100 - [(WT\ H_{SUB} - WT\ H_{PRÄ\text{-}OP}) / (WTH\ _{POST\text{-}OP} - WT\ H_{PRÄ\text{-}OP}) * 100]$$

MPE: Maximal Possible Effect
$WTH_{SUB}$: WEGZIEHSCHWELLE NACH SUBSTANZGABE
$WTh_{prä\text{-}op}$: Wegziehschwelle vor der Operation (Vortestmittelwert)
$WTh_{post\text{-}op}$: Wegziehschwelle nach der Operation und vor der Substanzgabe

Zur Signifikanzberechnung wird der Mann-Whitney-U- Test verwendet ($p < 0.05$). Bei dosisabhängigen Effekten wird der $ED_{50}$-Wert anhand einer Regressionsanalyse bestimmt.

**5. Ergebnisse:**

Die Ergebnisse sind in Tabelle 5 zusammengefaßt:

Tabelle 5 : Analgesieprüfung im Paw Incision Ratte

| Verbindung Nr. | Wert |
|---|---|
| 6 | 27% MPE (464 mg/kg) i.p. |

Gabapentin zeigt einen Wert von 66% MPE bei 100 mg/kg.

**Beispiel 8: Parenterale Applikationsform.**

38,5 g der Verbindung 4 werden in 1 l Wasser für Injektionszwecke bei Raumtemperatur gelöst und anschließend durch Zugabe von wasserfreier Glukose für Injektionszwecke auf isotone Bedingungen eingestellt.

**Patentansprüche**

1. Verwendung einer β-Thio-α-aminosäure der allgemeinen Formel I,

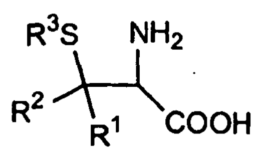

I

, worin

$R^1$ und $R^2$ jeweils unabhängig voneinander ausgewählt sind aus H; $C_{1-10}$-Alkyl, verzweigt oder unverzweigt, gesättigt oder ungesättigt, unsubstituiert oder ein- oder mehrfach substituiert; Benzyl, Aryl, $C_{3-8}$-Cycloalkyl oder Heteroaryl, jeweils unsubstituiert oder ein- oder mehrfach substituiert; oder

$R^1$ und $R^2$ zusammen einen $(CH_2)_{3-6}$-Ring bilden, gesättigt oder ungesättigt, substituiert oder unsubstituiert, in dem 0-2 C-Atome durch S, O oder $NR^4$, ersetzt sein können,

mit $R^4$ ausgewählt aus: H; $C_{1-10}$-Alkyl, gesättigt oder ungesättigt, verzweigt oder unverzweigt, einfach oder mehrfach substituiert oder unsubstituiert;

$R^3$ ausgewählt ist aus H; $C_{1-10}$-Alkyl, gesättigt oder ungesättigt, verzweigt oder unverzweigt, einfach oder

mehrfach substituiert oder unsubstituiert; $C_{3-8}$-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes $C_{1-3}$-Alkyl gebundenem Aryl, $C_{3-8}$-Cycloalkyl oder Heteroaryl, jeweils unsubstituiert oder einfach oder mehrfach substituiert;

in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen;

mit Ausnahme von Verbindungen, bei denen $R^1$, $R^2$ und $R^3$ gleichzeitig H sind oder $R^1$ und $R^2$ gleichzeitig $CH_3$ sind und $R^3$ Wasserstoff entspricht,

zur Herstellung eines Arzneimittels zur Behandlung von Schmerz, insbesondere von neuropathischem, chronischem oder akutem Schmerz, von Epilepsie und/oder von Migräne

oder

zur Herstellung eines Arzneimittels zur Behandlung von Hyperalgesie und Allodynie, insbesondere thermischer Hyperalgesie, mechanischer Hyperalgesie und Allodynie und Kälte Allodynie, oder von inflammatorischem oder postoperativem Schmerz

oder

zur Herstellung eines Arzneimittels zur Behandlung von Hitzewallungen, Beschwerden in der Postmenopause , Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

2. Verwendung nach Anspruch 1, **dadurch gekennzeichnet, daß** eine Thioaminosäure gemäß Formel I verwendet wird, worin

$R^1$ und $R^2$ jeweils unabhängig voneinander ausgewählt sind aus $C_{1-10}$-Alkyl, verzweigt oder unverzweigt, gesättigt oder ungesättigt, unsubstituiert oder ein- oder mehrfach substituiert; Benzyl, Aryl, $C_{3-8}$-Cycloalkyl oder Heteroaryl, jeweils unsubstituiert oder ein- oder mehrfach substituiert;

oder

$R^1$ und $R^2$ zusammen einen $(CH_2)_{3-6}$-Ring bilden, gesättigt oder ungesättigt, substituiert oder unsubstituiert, in dem 0-2 C-Atome durch S, O oder $NR^4$, ersetzt sein können.

3. Verwendung nach einem der Ansprüche 1 oder 2, **dadurch gekennzeichnet, daß** eine Thioaminosäure gemäß Formel I verwendet wird, worin

$R^1$ und $R^2$ jeweils unabhängig voneinander ausgewählt sind aus H; $C_{1-10}$-Alkyl, verzweigt oder unverzweigt, gesättigt oder ungesättigt, unsubstituiert oder ein- oder mehrfach substituiert; Phenyl oder Thiophenyl, jeweils unsubstituiert oder einfach substituiert; oder $C_{3-8}$-Cycloalkyl, unsubstituiert oder substituiert,

oder

$R^1$ und $R^2$ zusammen einen $(CH_2)_{3-6}$-Ring bilden, substituiert oder unsubstituiert, in dem 0-1 C-Atome durch S, O oder $NR_4$, ersetzt sein können.

4. Verwendung nach Anspruch 3, **dadurch gekennzeichnet, daß** eine Thioaminosäure gemäß Formel 1 verwendet wird, worin

einer der Reste $R^1$ und $R^2$ $C_{1-2}$-Alkyl, unsubstituiert oder ein- oder mehrfach substituiert; oder Phenyl, Thiophenyl, jeweils unsubstituiert oder einfach substituiert; oder $C_{3-8}$-Cycloalkyl unsubstituiert oder einfach substituiert; bedeutet und der andere der Reste $R^1$ und $R^2$ $C_{2-10}$-Alkyl, verzweigt oder unverzweigt, gesättigt oder ungesättigt, unsubstituiert oder ein- oder mehrfach substituiert; oder Phenyl bzw. Thiophenyl, jeweils unsubstituiert oder einfach substituiert; oder $C_{3-8}$-Cycloalkyl, jeweils unsubstituiert oder einfach substituiert; bedeutet,

oder

$R^1$ und $R^2$ zusammen Cyclopropyl, Cyclobutyl, Cyclopentyl, Cyclohexyl oder Cycloheptyl bilden, jeweils unsubstituiert oder einfach substituiert, wobei gegebenenfalls ein C-Atom im Ring durch S ersetzt ist.

5. Verwendung nach einem der Ansprüche 3 oder 4, **dadurch gekennzeichnet, daß** eine Thioaminosäure gemäß Formel I verwendet wird, worin

$R^1$ und $R^2$ jeweils unabhängig voneinander ausgewählt sind aus H; $C_{1-10}$-Alkyl, verzweigt oder unverzweigt, gesättigt oder ungesättigt, unsubstituiert oder ein- oder mehrfach substituiert; Phenyl oder Thiophenyl, jeweils unsubstituiert oder einfach mit $OCH_3$, $CH_3$, OH, SH, $CF_3$, F, Cl, Br oder I, substituiert; oder $C_{3-8}$-Cycloalkyl, unsubstituiert oder substituiert,

oder

einer der Reste $R^1$ und $R^2$ Methyl oder Ethyl; oder Phenyl, Thiophenyl, jeweils unsubstituiert oder einfach mit $OCH_3$, $CH_3$, OH, SH, $CF_3$, F, Cl, Br oder I, substituiert; oder $C_{3-8}$-Cycloalkyl unsubstituiert oder einfach substituiert; bedeutet und der andere der Reste $R^1$ und $R^2$ Ethyl, n-Propyl, i-Propyl, n-Butyl, i-Butyl, tert.-Butyl, Pentyl, Hexyl, Heptyl oder Octyl, verzweigt oder unverzweigt, gesättigt oder ungesättigt, unsubstituiert oder ein- oder mehrfach substituiert; oder Phenyl bzw. Thiophenyl, jeweils unsubstituiert oder einfach mit $OCH_3$, $CH_3$, OH, SH, $CF_3$, F, Cl, Br oder I, substituiert; oder Cyclopropyl, Cyclobutyl, Cyclopentyl, Cyclohexyl oder Cycloheptyl; bedeutet,

oder

$R^1$ und $R^2$ zusammen Cyclopropyl, Cyclobutyl oder Cyclopentyl bilden, wobei gegebenenfalls ein C-Atom im Ring durch S ersetzt ist.

**6.** Verwendung gemäß einem der Ansprüche 1 bis 5, **dadurch gekennzeichnet, daß** eine Thioaminosäure gemäß Formel I verwendet wird, worin

$R^3$ ausgewählt ist aus H; $C_{1-6}$-Alkyl, gesättigt oder ungesättigt, verzweigt oder unverzweigt, einfach oder mehrfach substituiert oder unsubstituiert; Phenyl oder Thiophenyl, unsubstituiert oder einfach substituiert; oder über gesättigtes $CH_3$-gebundenem Phenyl, unsubstituiert oder einfach substituiert.

**7.** Verwendung gemäß Anspruch 6, **dadurch gekennzeichnet, daß** eine Thioaminosäure gemäß Formel I verwendet wird, worin

$R^3$ ausgewählt ist aus $C_{1-6}$-Alkyl, gesättigt, unverzweigt und unsubstituiert; Phenyl oder Thiophenyl, einfach mit $OCH_3$, $CH_3$, OH, SH, $CF_3$, F, Cl, Br oder I, substituiert; oder über gesättigtes $CH_3$-gebundenem Phenyl, einfach mit $OCH_3$, $CH_3$, OH, SH, $CF_3$, F, Cl, Br oder I, substituiert.

**8.** Verwendung gemäß Anspruch 7, **dadurch gekennzeichnet, daß** eine Thioaminosäure gemäß Formel I verwendet wird, worin

$R^3$ ausgewählt ist aus Methyl, Ethyl, Propyl, n-Propyl, i-Propyl, Butyl, n-Butyl, i-Butyl, tert.-Butyl. Pentyl oder Hexyl.

**9.** Verwendung gemäß einem der Ansprüche 1 bis 8, **dadurch gekennzeichnet, daß** für die verwendete Thioaminosäure gemäß Formel I gilt, daß,

- wenn einer von $R^1$ oder $R^2$ Wasserstoff ist und $R^3$ Benzyl oder H ist, der andere von $R^1$ oder $R^2$ nicht Phenyl sein darf,
- wenn $R^1$ und $R^2$ zusammen Cyclopentyl bilden, $R^3$ nicht H sein darf,
- wenn einer von $R^1$ oder $R^2$ Wasserstoff und der andere von $R^1$ oder $R^2$ Phenyl ist, $R^3$ nicht substituiertes oder unsubstituiertes Benzyl sein darf oder
- wenn einer von $R^1$ oder $R^2$ Wasserstoff und der andere von $R^1$ oder $R^2$ Methyl ist, $R^3$ nicht H sein darf.

**10.** Verwendung gemäß einem der Ansprüche 1 bis 9, **dadurch gekennzeichnet, daß** eine Thioaminosäure ausgewählt aus der folgenden Gruppe verwendet wird:

- 2-Amino-3-mercapto-3-methyl-pentansäure
- 2-Amino-3-mercapto-3-methyl-hexansäure
- 2-Amino-3-mercapto-3-methyl-heptansäure
- 2-Amino-3-mercapto-3-methyl-octansäure
- 2-Amino-3-mercapto-3-methyl-nonansäure
- 2-Amino-3-mercapto-3-methyl-decansäure
- 2-Amino-3-ethyl-3-mercapto-pentansäure
- Amino-(1-mercapto-cyctopentyl)essigsäure
- Amino-3-ethyl-3-mercapto-hexansäure
- 2-Amino-3-mercapto-3-methyl-decansäure
- 2-Amino-3-mercapto-3-methyl-nonansäure
- 2-Amino-B-mercapto-3-methyl-octansäure
- 2-Amino-3-ethylsulfanyl-3-methyl-octansäure

- 2-Amino-3-benzylsultanyl-3-methyl-octansäure
- 2-Amino-3-mercapto-3-propyl-3-hexansäure
- Amino-(1-mercapto-cycloheptyi)-essigsäure
- 2-Amino-3-mercapto-3-propyl-3-hexansäure
- Amino-(1-mercapto-cycloheptyl)-essigsäure.
- 2-Amino-3-ethylsulfanyl-3-methyl-nonansäure
- 2-Amino-3-methyl-3-propylsulfanyl-nonansäure
- 2-Amino-3-hexylsulfanyl-3-methyl-nonansäure
- 2-Amino-3-benzylsulfanyl-3-methyl-nonansäure
- 2-Amino-3-benzylsulfanyl-,3-methyl-decansäue
- 2-Amino-3-ethylsulfanyl-3-methyl-decansäure
- 2-Amino-3-cyclopropyl-3-(4-fluoro-phenyl)-3-mercapto-propansäure
- 2-Amino-3-cyclopropyl-3-mercapto-butansäure
- 2-Amino-3-cyclobutyl-3-mercapto-butansäure
- 2-Amino-3-cyclohexyl-3-mercapto-butansäure
- 2-Amino-3-mercapto-3-thiophen-2-yl-butansäure
- 2-Amino-3-ethyl-3-mercapto-heptansäure
- Amino-(1-mercapto-cyplohexyl)- ethansäure
- Amino-(1-morcapto-3-methyl-cyclohexyl)-ethansäure
- Amino-(1-mercapto-2-methyl-cyclohexyl)-ethansäure
- Amino-(1-mercapto-4-methyl-cyclohexyl)-ethansäure
- Amino-(4-mercapto-tetrahydro-thiopyran-4-yl)-ethansäure
- 2-Amino-3-mercapto-3,4-dimethyl-pentansäur
- 2-Amino-3-mercapto-3,4-dimethyl-hexansäure

in Form ihrer Razemate; Enantiomere, Diastereomere, Mischungen ihrer Enantiomere oder Diastereomere oder eines einzelnen Enantiomers oder Diastereomers; in Form Ihrer physiologisch verträglichen sauren und basischen Salze bzw. Salze mit Kationen bzw. Basen oder mit Anionen bzw. Säuren oder in Form der freien Säuren oder Basen.

**11.** Verwendung von Verbindungen gemäß Anspruch 10, **dadurch gekennzeichnet, dass** die Verbindungen in Form des Hydrochlorids vorliegen.

**12.** Verwendung gemäß einem der Ansprüche 1 bis 11. **dadurch gekennzeichnet, daß** mindestens eine verwendete Thioaminosäure als reines Diastereomer und/oder Enantiomer, als Razemat oder als nicht-äquimolare oder äquimolare Mischung der Diastereomere und/oder Enantiomere vorliegt.

**13.** β-Thio-a-aminosäure der allgemeinen Formel I,

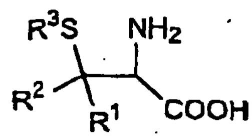

I

, worin

einer der Reste $R^1$ und $R^2$ $C_{1-6}$-Alkyl, gesättigt oder ungesättigt, verzweigt oder unverzweigt, einfach oder mehrfach substituiert oder unsubstituiert; bedeutet und der andere der Reste $R^1$ und $R^2$ $C_{3-10}$-Alkyl, gesättigt oder ungesättigt, verzweigt oder unverzweigt, einfach oder mehrfach mit F, Cl, Br, 1, $NH_2$, SH oder OH substituiert oder unsubstituiert; oder Phenyl, Thiophenyl oder $C_{3-8}$-Cycloalkyl, jeweils unsubstituiert oder einfach oder mehrfach substituiert; bedeutet,

und

$R^3$ ausgewählt ist aus H; $C_{1-10}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert: oder über gesättigtes oder ungesättigtes $C_{1-3}$-Alkyl gebundenem Aryl, $C_{3-8}$-Cycloalkyl oder Heteroaryl, jeweils

unsubstituiert oder einfach oder mehrfach substituiert;
in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen.

**14.** Thioaminosäure gemäß Anspruch 13, **dadurch gekennzeichnet, daß**
einer der Reste $R^1$ und $R^2$ $C_{1-2}$-Alkyl, einfach oder mehrfach substituiert oder unsubstituiert bedeutet und der andere der Reste $R^1$ und $R^2$ $C_{3-10}$-Alkyl, gesättigt oder ungesättigt, verzweigt oder unverzweigt, einfach oder mehrfach mit F, Cl, Br, I, $NH_2$. SH oder OH substituiert oder unsubstituiert; oder Phenyl oder Thiophenyl, jeweils unsubstituiert oder einfach substituiert: oder Cyclopropyl, Cyclobutyl, Cyclopentyl, Cyclohexyl oder Cycloheptyl; bedeutet

**15.** Thioaminosäure gemäß Anspruch 14, **dadurch gekennzeichnet, daß**
einer der Reste $R^1$ und $R^2$ Methyl oder Ethyl bedeutet und der andere der Reste $R^1$ und $R^2$ $C_{3-8}$-Alkyl, gesättigt oder ungesättigt, verzweigt oder unverzweigt, einfach oder mehrfach mit F. Cl, Br, I, $NH_2$, SH oder OH substituiert oder unsubstituiert; oder Phenyl oder Thiophenyl, jeweils unsubstituiert oder einfach mit $OCH_3$. $CH_3$. OH, SH, $CF_3$, F, Cl, Br oder I, substituiert; oder Cyclopropyl, Cyclobutyl, Cyclopentyl, Cyclohexyl oder Cycloheptyl; bedeutet.

**16.** Thioaminosäure gemäß Anspruch 15, **dadurch gekennzeichnet, daß**
einer der Reste $R^1$ und $R^2$ Methyl oder Ethyl bedeutet und der andere der Reste $R^1$ und $R^2$ Propyl, n-Propyl, i-Propyl. Butyl, n-Butyl, i-Butyl, tert.-Butyl, Pentyl, Hexyl, Heptyl oder Octyl; bedeutet.

**17.** Thioaminosäure gemäß einem der Ansprüche 13 bis 16, **dadurch gekennzeichnet, daß**
$R^3$ ausgewählt ist aus H; $C_{1-6}$-Alkyl, gesättigt oder ungesättigt, verzweigt oder unverzweigt, einfach oder mehrfach substituiert oder unsubstituiert; Phenyl oder Thiophenyl, unsubstituiert oder einfach substituiert; oder über gesättigtes $CH_3$-gebundenem Phenyl, unsubstituiert oder einfach substituiert.

**18.** Thioaminosäure gemäß Anspruch 17, **dadurch gekennzeichnet, daß**
$R^3$ ausgewählt ist aus $C_{1-6}$-Alkyl, gesättigt, unverzweigt und unsubstituiert; Phenyl oder Thiophenyl, einfach mit $OCH_3$, $CH_3$, OH, SH, $CF_3$, F, Cl, Br oder I, substituiert; oder über gesättigtes $CH_3$-gebundenem Phenyl einfach mit $OCH_3$, $CH_3$, OH, SH, $CF_3$, F, Cl, Br oder I, substituiert.

**19.** Thioaminosäure gemäß Anspruch 18, **dadurch gekennzeichnet, daß**
$R^3$ ausgewählt ist aus Methyl, Ethyl, Propyl, n-Propyl, i-Propyl, Butyl, n-Butyl, i-Butyl, tert.-Butyl, Pentyl oder Hexyl.

**20.** Thioaminosäure gemäß einem der Ansprüche 13 bis 19, **dadurch gekennzeichnet, daß** sie ausgewählt ist aus der folgenden Gruppe:

- 2-Amino-3-mercapto-3-methyl-hexansäure
- 2-Amino-3-mercapto-3-methyl-heptansäure
- 2-Amino-3-mercapto-3-methyl-octansäure
- 2-Amino-3-mercapto-3-methyl-nonansäure
- 2-Amino-3-mercapto-3-methyl-decansäure
- Amino-3-ethyl-3-mercapto-hexansäure
- 2-Amino-3-mercapto-3-methyl-decansäure
- 2-Amino-3-mercapto-3-methyl-nonansäure
- -2-Amino-3-mercapto-3-methyl-octansäure
- 2-Amino-3-ethylsulfanyl-3-methyl-octansäure
- 2-Amino-3-benrylsulfanyl-3-methyl-octansäure
- 2-Amino-3-mercapto-3-propyl-3-hexansäure
- Amino-(1-mercapto-cycloheptyl)-essigsäure
- 2-Amino-3-mercapto-3-propyl-3-hexansaure
- 2-Amino-3-ethylsulfanyl-3-methyl-nonansäure
- 2-Amino-3-methyl-3-propylsulfanyl-nonansäure
- 2-Amino-3-hexylsulfanyl-3-methyl-nonansäure

- 2-Amino-3-benzylsulfanyl-3-methyl-nonansäure
- 2-Amino-3-benzylsulfanyl-3-methyl-decansäure
- 2-Amino-3-ethytsulfanyl-3-methyl-decansäure
- 2-Amino-3-cyclopropyl-3-mercapto-butansäure
- 2-Amino-3-cyclobutyl-3-mercapto-butansäure
- 2-Amino-3-cyclohexyl-3-mercapto-butansäure
- 2-Amino-3-mercapto-3-thiophen-2-yl-butansäure
- 2-Amino-3-ethyl-3-mercapto-heptansäure
- 2-Amino-3-mercapto-3.4-dimethyl-pentansäure
- 2-Amino-3-mercapto-3,4-dimethyl-hexansäure

in Form ihrer Razemate; Enantiomere, Diastereomere, Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen.

**21.** Thioaminosäuren gemäß Anspruch 20, **dadurch gekennzeichnet, dass** die Verbindungen in Form des Hydrochlorids vorliegen.

**22.** Arzneimittel enthaltend wenigstens eine Thioaminosäure gemäß einem der Ansprüche 13 bis 21, sowie gegebenenfalls geeignete Zusatz- und/oder Hilfsstoffe und/oder gegebenenfalls weitere Wirkstoffe.

**23.** Arzneimittel gemäß Anspruch 22, **dadurch gekennzeichnet, daß** eine enthaltene Thioaminosäure gemäß einem der Ansprüche 13 bis 21, als reines Diastereomer und/oder Enantiomer, als Razemat oder als nicht-äquimolare oder äquimolare Mischung der Diastereomere und/oder Enantiomere vorliegt.

**24.** Verwendung einer Thioaminosäure gemäß einem der Ansprüche 13 bis 21
zur Herstellung eines Arzneimittels zur Behandlung von Schmerz, insbesondere von neuropathischem, chronischem oder akutem Schmerz, von Epilepsie und/oder von Migräne
oder
zur Herstellung eines Arzneimittels zur Behandlung von Hyperalgesie und Allodynie, insbesondere thermischer Hyperalgesie, mechanischer Hyperalgesie und Allodynie und Kälte Allodynie, oder von inflammatorischem oder postoperativem Schmerz
oder
zur Herstellung eines Arzneimittels zur Behandlung von Hitzewallungen, Beschwerden in der Postmenopause , Amyotropischer Lateraler Sklerose (ALS), Reflex Sympasthetic Dystrophy (RSD), Spastischer Lähmung, Restless Leg Syndrom, erworbenem Nystagmus: psychatrischen bzw. neuropathologischen Störungen, wie bipolaren Störungen, Anxiety, Panikanfällen, Stimmungsschwankungen, manischem Verhalten. Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

**25.** Verfahren zur Herstellung einer Thioaminsäure gemäß einem der Ansprüche 13 bis 21 mit folgenden Schritten:

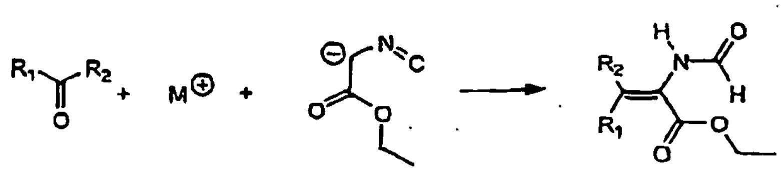

2

3

Deprotonierung des Isocyanessigsäurethylesters mit Basen, vorzugsweise Butyllithium, Natriumhydrid oder Kalium.-tert.butylat, und anschließende Umsetzung mit Ketonen der allgemeinen Formel 2 in Tetrahydrofuran führt zu (E.Z)-2-Fomylaminoacrylsäureethylestern der allgemeinen Formel 3,

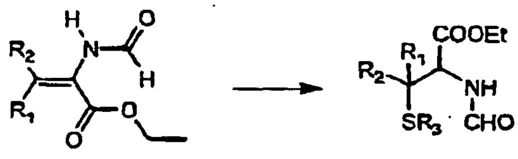

3 4

Umsetzung von (E,Z)-2-Formylaminoacrylsäureethylestern der allgemeinen Formel 3 mit $P_4S_{10}$ in Toluol oder mit Mercaptanen der allgemeinen Formel $R_3SH$ in Gegenwart von Butyllithium in Toluol, was zu Formylamino-Ethylester der allgemeinen Formel 4 führt,

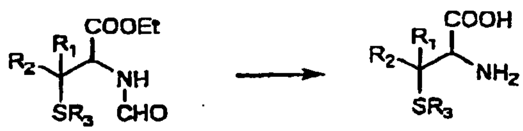

4 1

Reaktion der Formylamino-Ethylester der allgemeinen Formel 4 mit Säure, vorzugsweise Salzsäure, was zu den Thio-Aminosäuren der allgemeinen Formel 1 bzw. I gemäß einem der Ansprüche 12 bis 19 führt, gegebenenfalls gefolgt oder unterbrochen von Diastereomerentrennung auf geeigneter Stufe mittels HPLC, Säulenchromatographie oder Kristallisation bzw. gefolgt von Enantiomerentrennung mittels HPLC, Säulenchromatographie oder Kristallisation,
wobei $R^1$ bis $R^3$ die Bedeutung gemäß Anspruch 12 haben oder einem mit einer geeigneten Schutzgruppe geschützten entsprechenden Rest entsprechen.

## Claims

**1.** Use of a β-thio-α-amino acid of the general formula I,

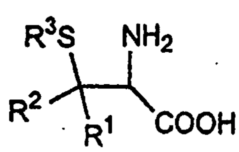

I

wherein
$R^1$ and $R^2$ are in each case selected independently of one another from H; $C_{1-10}$-alkyl that is branched or unbranched, saturated or unsaturated, unsubstituted or singly or multiply substituted; benzyl, aryl, $C_{3-8}$-cycloalkyl or heteroaryl, in each case unsubstituted or singly or multiply substituted; or
$R^1$ and $R^2$ together form a $(CH_2)_{3-6}$ ring, saturated or unsaturated, substituted or unsubstituted, in which 0-2 C atoms may be replaced by S, O or $NR^4$,
where $R^4$ is selected from H; $C_{1-10}$-alkyl that is saturated or unsaturated, branched or unbranched, singly or multiply substituted, or unsubstituted;
$R^3$ is selected from H; $C_{1-10}$-alkyl that is saturated or unsaturated, branched or unbranched, singly or multiply substituted or unsubstituted;
$C_{3-8}$-cycloalkyl that is saturated or unsaturated, unsubstituted or singly or multiply substituted; aryl or heteroaryl, in each case unsubstituted or singly or multiply substituted; or aryl, $C_{3-8}$-cycloalkyl or heteroaryl bound by saturated or unsaturated $C_{1-3}$-alkyl and in each case unsubstituted or singly or multiply substituted;
in the form of their racemates, enantiomers, diastereomers, in particular mixtures of their enantiomers or diastereomers, or of an individual enantiomer or diastereomer; in the form of their physiologically compatible acidic and basic salts and/or salts with cations and/or bases or with anions and/or acids or in the form of the free acids or bases;

with the exception of the compounds in which $R^1$, $R^2$ and $R^3$ are simultaneously H, or $R^1$ and $R^2$ are simultaneously $CH_3$ and $R^3$ corresponds to hydrogen,
for the production of a medicament for the treatment of pain, in particular neuropathic, chronic or acute pain, epilepsy and/or migraine
or
for the production of a medicament for the treatment of hyperalgesia and allodynia, in particular thermal hyperalgesia, mechanical hyperalgesia and allodynia and cold-induced allodynia, or inflammatory or postoperative pain
or
for the production of a medicament for the treatment of hot flushes, post-menopausal symptoms, amyotropic lateral sclerosis (ALS), reflex sympathetic dystrophy (RSD), spastic paralysis, restless leg syndrome, acquired nystagmus; psychiatric or neuropathological disorders such as bipolar disorders, anxiety, panic attacks, mood fluctuations, manic behaviour, depression, manic-depressive behaviour; painful diabetic neuropathy, symptoms and pain due to multiple sclerosis or Parkinson's disease, neurodegenerative diseases such as Alzheimer's disease, Huntington's disease, Parkinson's disease and epilepsy; gastrointestinal lesions; erythromelalgic or post-poliomyelitic pain, trigeminal or post-herpes neuralgia; or as an anticonvulsant, analgesic or anxiolytic.

**2.** Use according to claim 1, **characterised in that** a thioamino acid according to formula I is used, wherein
$R^1$ and $R^2$ are in each case selected independently of one another from $C_{1-10}$-alkyl that is branched or unbranched, saturated or unsaturated, unsubstituted or singly or multiply substituted; from benzyl, aryl, $C_{3-8}$-cycloalkyl or heteroaryl, in each case unsubstituted or singly or multiply substituted,
or
$R^1$ and $R^2$ together form a $(CH_2)_{3-6}$ ring that is saturated or unsaturated, substituted or unsubstituted, in which 0-2 C atoms may be replaced by S, O or $NR^4$.

**3.** Use according to one of claims 1 and 2, **characterised in that** a thioamino acid according to formula I is used, wherein
$R^1$ and $R^2$ are in each case selected independently of one another from H; $C_{1-10}$-alkyl that is branched or unbranched, saturated or unsaturated, unsubstituted or singly or multiply substituted; from phenyl or thiophenyl, in each case unsubstituted or singly substituted; or from $C_{3-8}$-cycloalkyl that is unsubstituted or substituted,
or
$R^1$ and $R^2$ together form a $(CH_2)_{3-6}$ ring that is substituted or unsubstituted, in which 0-1 C atoms may be replaced by S, or $NR_4$.

**4.** Use according to claim 3, **characterised in that** a thioamino acid according to formula I is used, wherein
one of the radicals $R^1$ and $R^2$ denotes $C_{1-2}$-alkyl that is unsubstituted or singly or multiply substituted; or denotes phenyl, thiophenyl, in each case unsubstituted or singly substituted; or denotes $C_{3-8}$-cycloalkyl that is unsubstituted or singly substituted; and the other of the radicals $R^1$ and $R^2$ denotes $C_{2-10}$-alkyl, which is branched or unbranched, saturated or unsaturated, unsubstituted or singly or multiply substituted; or denotes phenyl or thiophenyl, in each case unsubstituted or singly substituted; or denotes $C_{3-8}$-cycloalkyl, in each case unsubstituted or singly substituted,
or
$R^1$ and $R^2$ together form cyclopropyl, cyclobutyl, cyclopentyl, cyclohexyl or cycloheptyl in each case unsubstituted or singly substituted, in which a C atom in the ring is optionally replaced by S.

**5.** Use according to one of claims 3 or 4, **characterised in that** a thioamino acid according to formula I is used, wherein
$R^1$ and $R^2$ are in each case selected independently of one another from H; $C_{1-10}$-alkyl that is branched or unbranched, saturated or unsaturated, unsubstituted or singly or multiply substituted; from phenyl or thiophenyl, in each case unsubstituted or singly substituted with $OCH_3$, $CH_3$, OH, SH, $CF_3$, F, Cl, Br or I; or from $C_{3-8}$-cycloalkyl that is unsubstituted or substituted,
or
one of the radicals $R^1$ and $R^2$ denotes methyl or ethyl; or denotes phenyl, thiophenyl, in each case unsubstituted or singly substituted with $OCH_3$, $CH_3$, OH, SH, $CF_3$, F, Cl, Br or I; or denotes $C_{3-8}$-cycloalkyl that is unsubstituted or singly substituted; and the other of the radicals $R^1$ and $R^2$ denotes ethyl, n-propyl, i-propyl, n-butyl, i-butyl, tert.-butyl, pentyl, hexyl, heptyl or octyl, which is branched or unbranched, saturated or unsaturated, unsubstituted or singly or multiply substituted; or denotes phenyl or thiophenyl, in each case unsubstituted or singly substituted with $OCH_3$, $CH_3$, OH, SH, $CF_3$, F, Cl, Br or I; or denotes cyclopropyl, cyclobutyl, cyclopentyl, cyclohexyl or cycloheptyl;
or

$R^1$ and $R^2$ together form cyclopropyl, cyclobutyl or cyclopentyl, in which a C atom in the ring is optionally replaced by S.

6. Use according to one of claims 1 to 5, **characterised in that** a thioamino acid according to formula I is used wherein $R^3$ is selected from H; $C_{1-6}$-alkyl that is saturated or unsaturated, branched or unbranched, singly or multiply substituted or unsubstituted; phenyl or thiophenyl that is unsubstituted or singly substituted; or phenyl bound via saturated $CH_3$, that is unsubstituted or singly substituted.

7. Use according to claim 6, **characterised in that** a thioamino acid according to formula I is used, wherein $R^3$ is selected from $C_{1-6}$-alkyl that is saturated, unbranched and unsubstituted; phenyl or thiophenyl, that is singly substituted with $OCH_3$, $CH_3$, OH, SH, $CF_3$, F, Cl, Br or I; or phenyl bound via saturated $CH_3$, that is singly substituted with $OCH_3$, $CH_3$, OH, SH, $CF_3$, F, Cl, Br or I.

8. Use according to claim 7, **characterised in that** a thioamino acid according to formula I is used, wherein $R^3$ is selected from methyl, ethyl, propyl, n-propyl, i-propyl, butyl, n-butyl, i-butyl, tert.-butyl, pentyl or hexyl.

9. Use according to one of claims 1 to 8, **characterised in that** the following applies to the thioamino acid according to formula I that is used:

- if one of $R^1$ or $R^2$ is hydrogen and $R^3$ is benzyl or H, the other of $R^1$ or $R^2$ may not be phenyl,
- if $R^1$ and $R^2$ together form cyclopentyl, $R^3$ may not be H,
- if one of $R^1$ or $R^2$ is hydrogen and the other of $R^1$ or $R^2$ is phenyl, $R^3$ may not be substituted or unsubstituted benzyl, or
- if one of $R^1$ or $R^2$ is hydrogen and the other of $R^1$ or $R^2$ is methyl, $R^3$ may not be H.

10. Use according to one of claims 1 to 9, **characterised in that** a thioamino acid selected from the following group is used:

- 2-amino-3-mercapto-3-methylpentanoic acid
- 2-amino-3-mercapto-3-methylhexanoic acid
- 2-amino-3-mercapto-3-methylheptanoic acid
- 2-amino-3-mercapto-3-methyloctanoic acid
- 2-amino-3-mercapto-3-methylnonanoic acid
- 2-amino-3-mercapto-3-methyldecanoic acid
- 2-amino-3-ethyl-3-mercaptopentanoic acid
- amino-(1-mercaptocyclopentyl)acetic acid
- amino-3-ethyl-3-mercaptohexanoic acid
- 2-amino-3-mercapto-3-methyldecanoic acid
- 2-amino-3-mercapto-3-methylnonanoic acid
- 2-amino-3-mercapto-3-methyloctanoic acid
- 2-amino-3-ethylsulfanyl-3-methyloctanoic acid
- 2-amino-3-benzylsulfanyl-3-methyloctanoic acid
- 2-amino-3-mercapto-3-propyl-3-hexanoic acid
- amino-(1-mercaptocycloheptyl)acetic acid
- 2-amino-3-mercapto-3-propyl-3-hexanoic acid
- amino-(1-mercaptocycloheptyl)acetic acid
- 2-amino-3-ethylsulfanyl-3-methylnonanoic acid
- 2-amino-3-methyl-3-propylsulfanylnonanoic acid
- 2-amino-3-hexylsulfanyl-3-methylnonanoic acid
- 2-amino-3-benzylsulfanyl-3-methylnonanoic acid
- 2-amino-3-benzylsulfanyl-3-methyldecanoic acid
- 2-amino-3-ethylsulfanyl-3-methyldecanoic acid
- 2-amino-3-cyclopropyl-3-(4-fluorophenyl)-3-mercaptopropanoic acid
- 2-amino-3-cyclopropyl-3-mercaptobutanoic acid
- 2-amino-3-cyclobutyl-3-mercaptobutanoic acid
- 2-amino-3-cyclohexyl-3-mercaptobutanoic acid
- 2-amino-3-mercapto-3-thiophen-2-yl-butanoic acid
- 2-amino-3-ethyl-3-mercaptoheptanoic acid

- amino-(1-mercaptocyclohexyl)-ethanoic acid
- amino- (1-mercapto-3-methylcyclohexyl)-ethanoic acid
- amino-(1-mercapto-2-methylcyclohexyl)-ethanoic acid
- amino-(1-mercapto-4-methylcyclohexyl)-ethanoic acid
- amino-(4-mercaptotetrahydrothiopyran-4-yl)-ethanoic acid
- 2-amino-3-mercapto-3,4-dimethylpentanoic acid
- 2-amino-3-mercapto-3,4-dimethylhexanoic acid

in the form of their racemates, enantiomers, diastereomers, in particular mixtures of their enantiomers or diastereomers, or of an individual enantiomer or diastereomer; in the form of their physiologically compatible acidic and basic salts or salts with cations and/or bases or with anions or acids or in the form of the free acids or bases.

**11.** Use of compounds according to claim 10, **characterised in that** the compounds are in the form of the hydrochloride.

**12.** Use according to one of claims 1 to 11, **characterised in that** at least one thioamino acid that is used is present as pure diastereomer and/or enantiomer, as racemate or as a non-equimolar or equimolar mixture of the diastereomers and/or enantiomers.

**13.** β-thio-α-amino acid of the general formula I,

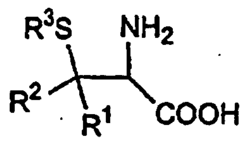,

I

wherein

one of the radicals $R^1$ and $R^2$ denotes $C_{1-6}$-alkyl that is saturated or unsaturated, branched or unbranched, singly or multiply substituted or unsubstituted; and the other of the radicals $R^1$ and $R^2$ denotes $C_{3-10}$-alkyl that is saturated or unsaturated, branched or unbranched, singly or multiply substituted or unsubstituted with F, Cl, Br, I, $NH_2$, SH or OH; or denotes phenyl, thiophenyl or $C_{3-8}$-cycloalkyl, in each case unsubstituted or singly or multiply substituted,

and

$R^3$ is selected from H; $C_{1-10}$-alkyl that is saturated or unsaturated, branched or unbranched, singly or multiply substituted, or unsubstituted; $C_{3-8}$-cycloalkyl that is saturated or unsaturated, unsubstituted or singly or multiply substituted; aryl or heteroaryl, in each case unsubstituted or singly or multiply substituted; or denotes aryl, $C_{3-8}$-cycloalkyl or heteroaryl, bound via saturated or unsaturated $C_{1-3}$-alkyl, in each case unsubstituted or singly or multiply substituted,

in the form of their racemates; enantiomers, diastereomers, in particular mixtures of their enantiomers or diastereomers, or of an individual enantiomer or diastereomer; in the form of their physiologically compatible acidic and basic salts or salts with cations or bases or with anions or acids, or in the form of the free acids or bases.

**14.** Thioamino acid according to claim 13, **characterised in that**

one of the radicals $R^1$ and $R^2$ denotes $C_{1-2}$-alkyl that is singly or multiply substituted or unsubstituted and the other of the radicals $R^1$ and $R^2$ denotes $C_{3-10}$-alkyl that is saturated or unsaturated, branched or unbranched, singly or multiply substituted or unsubstituted with F, Cl, Br, I, $NH_2$, SH or OH; or phenyl or thiophenyl, in each case unsubstituted or singly substituted; or denotes cyclopropyl, cyclobutyl, cyclopentyl, cyclohexyl or cycloheptyl.

**15.** Thioamino acid according to claim 14, **characterised in that**

one of the radicals $R^1$ and $R^2$ denotes methyl or ethyl and the other of the radicals $R^1$ and $R^2$ denotes $C_{3-8}$-alkyl that is saturated or unsaturated, branched or unbranched, singly or multiply substituted or unsubstituted with F, Cl, Br, I, $NH_2$, SH or OH; or phenyl or thiophenyl, in each case unsubstituted or singly substituted with $OCH_3$, $CH_3$, OH, SH, $CF_3$, F, Cl, Br or I; or denotes cyclopropyl, cyclobutyl, cyclopentyl, cyclohexyl or cycloheptyl.

**16.** Thioamino acid according to claim 15, **characterised in that**

one of the radicals $R^1$ and $R^2$ denotes methyl or ethyl, and the other of the radicals $R^1$ and $R^2$ denotes propyl, n-propyl, i-propyl, butyl, n-butyl, i-butyl, tert.-butyl, pentyl, hexyl, heptyl or octyl.

**17.** Thioamino acid according to one of claims 13 to 16, **characterised in that**
$R_3$ is selected from H; $C_{1-6}$-alkyl that is saturated or unsaturated, branched or unbranched, singly or multiply substituted or unsubstituted; phenyl or thiophenyl that is unsubstituted or singly substituted; or phenyl bound via saturated $CH_3$, that is unsubstituted or singly substituted.

**18.** Thioamino acid according to claim 17, **characterised in that**
$R^3$ is selected from $C_{1-6}$-alkyl that is saturated, unbranched and unsubstituted; phenyl or thiophenyl that is singly substituted with $OCH_3$, $CH_3$, OH, SH, $CF_3$, F, Cl, Br or I; or phenyl bound via saturated $CH_3$ that is singly substituted with $OCH_3$, $CH_3$, OH, SH, $CF_3$, F, Cl, Br or I.

**19.** Thioamino acid according to claim 18, **characterised in that**
$R^3$ is selected from methyl, ethyl, propyl, n-propyl, i-propyl, butyl, n-butyl, i-butyl, tert.-butyl, pentyl or hexyl.

**20.** Thioamino acid according to one of claims 13 to 19, **characterised in that** it is selected from the following group:

- 2-amino-3-mercapto-3-methylhexanoic acid
- 2-amino-3-mercapto-3-methylheptanoic acid
- 2-amino-3-mercapto-3-methyloctanoic acid
- 2-amino-3-mercapto-3-methylnonanoic acid
- 2-amino-3-mercapto-3-methyldecanoic acid
- amino-3-ethyl-3-mercaptohexanoic acid
- 2-amino-3-mercapto-3-methyldecanoic acid
- 2-amino-3-mercapto-3-methylnonanoic acid
- 2-amino-3-mercapto-3-methyloctanoic acid
- 2-amino-3-ethylsulfanyl-3-methyloctanoic acid
- 2-amino-3-benzylsulfanyl-3-methyloctanoic acid
- 2-amino-3-mercapto-3-propyl-3-hexanoic acid
- amino-(1-mercaptocycloheptyl)acetic acid
- 2-amino-3-mercapto-3-propyl-3-hexanoic acid
- 2-amino-3-ethylsulfanyl-3-methylnonanoic acid
- 2-amino-3-methyl-3-propylsulfanylnonanoic acid
- 2-amino-3-hexylsulfanyl-3-methylnonanoic acid
- 2-amino-3-benzylsulfanyl-3-methylnonanoic acid
- 2-amino-3-benzylsulfanyl-3-methyldecanoic acid
- 2-amino-3-ethylsulfanyl-3-methyldecanoic acid
- 2-amino-3-cyclopropyl-3-mercaptobutanoic acid
- 2-amino-3-cyclobutyl-3-mercaptobutanoic acid
- 2-amino-3-cyclohexyl-3-mercaptobutanoic acid
- 2-amino-3-mercapto-3-thiophen-2-yl-butanoic acid
- 2-amino-3-ethyl-3-mercaptoheptanoic acid
- 2-amino-3-mercapto-3,4-dimethylpentanoic acid
- 2-amino-3-mercapto-3,4-dimethylhexanoic acid

in the form of their racemates; enantiomers, diastereomers, mixtures of their enantiomers or diastereomers or of an individual enantiomer or diastereomer; in the form of their physiologically compatible acidic and basic salts or salts with cations or bases or with anions or acids, or in the form of the free acids or bases.

**21.** Thioamino acids according to claim 20, **characterised in that** the compounds are in the form of the hydrochloride.

**22.** Medicament containing at least one thioamino acid according to one of claims 13 to 21, as well as optionally suitable additives and/or auxiliary substances and/or optionally further active constituents.

**23.** Medicament according to claim 22, **characterised in that** a contained thioamino acid according to one of claims 13 to 21 is present as pure diastereomer and/or enantiomer, as racemate, or as a non-equimolar or equimolar mixture of the diastereomers and/or enantiomers.

**24.** Use of a thioamino acid according to one of claims 13 to 21
for the production of a medicament for treating pain, in particular neuropathic, chronic or acute pain, epilepsy and/or migraine
or
for the production of a medicament for the treatment of hyperalgesia and allodynia, in particular thermal hyperalgesia, mechanical hyperalgesia and allodynia and cold-induced allodynia, or inflammatory or postoperative pain
or
for the production of a medicament for the treatment of hot flushes, post-menopausal symptoms, amyotropic lateral sclerosis (ALS), reflex sympathetic dystrophy (RSD), spastic paralysis, restless leg syndrome, acquired nystagmus; psychiatric or neuropathological disorders such as bipolar disorders, anxiety, panic attacks, mood fluctuations, manic behaviour, depression, manic-depressive behaviour; painful diabetic neuropathy, symptoms and pain due to multiple sclerosis or Parkinson's disease, neurodegenerative diseases such as Alzheimer's disease, Huntington's disease, Parkinson's disease and epilepsy; gastrointestinal damage; erythromelalgic or post-poliomyelitic pain, trigeminal or post-herpes neuralgia; or as an anticonvulsant, analgesic or anxiolytic.

**25.** Process for the production of a thioamino acid according to one of claims 13 to 21, comprising the following steps:

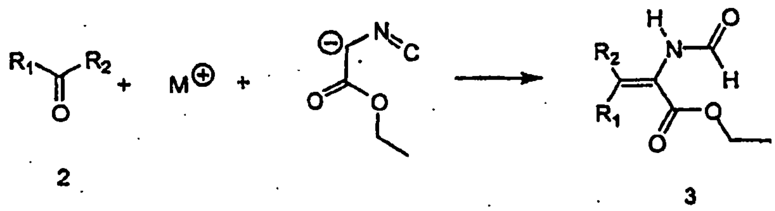

2 3

Deprotonation of the isocyanoacetic acid ethyl esters with bases, preferably butyllithium, sodium hydride or potassium tert.-butylate followed by reaction with ketones of the general formula 2 in tetrahydrofuran leads to (E,Z)-2-formylaminoacrylic acid ethyl esters of the general formula 3,

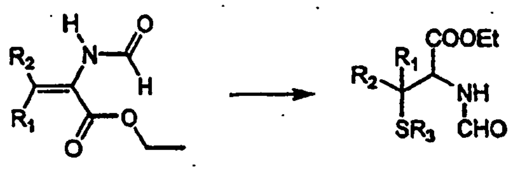

3 4

reaction of (E,Z)-2-formylaminoacrylic acid ethyl esters of the general formula 3 with $P_4S_{10}$ in toluene or with mercaptans of the general formula $R_3SH$ in the presence of butyllithium in toluene, which leads to formylamino ethyl esters of the general formula 4

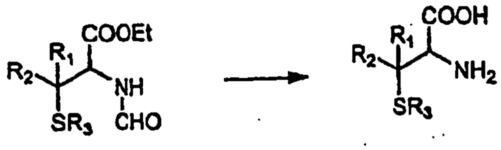

4 1

reaction of the formylamino ethyl esters of the general formula 4 with acid, preferably hydrochloric acid, which leads to the thioamino acids of the general formula 1 or I according to one of claims 12 to 19, optionally followed or interrupted by separation of the diastereomers at a suitable stage by means of HPLC, column chromatography

or crystallisation, or followed by separation of the enantiomers by means of HPLC, column chromatography or crystallisation,
wherein R1 to R3 have the meanings according to claim 12 or correspond to a corresponding radical protected with a suitable protective group.

## Revendications

**1.** Utilisation d'un β-thio-α-aminoacide de formule générale I,

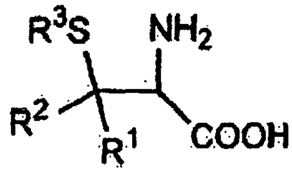

I

dans laquelle $R^1$ et $R^2$ sont tous deux choisis, indépendamment l'un de l'autre, entre H ; un reste alkyle en $C_1$ à $C_{10}$, ramifié ou non ramifié, saturé ou non saturé, non substitué ou substitué une ou plusieurs fois ; benzyle, aryle, cycloalkyle en $C_3$ à $C_8$ ou hétéroaryle, chacun non substitué ou substitué une ou plusieurs fois ; ou bien

$R^1$ et $R^2$ forment ensemble un noyau $(CH_2)_{3-6}$, saturé ou non saturé, substitué ou non substitué, dans lequel 0 à 2 atomes de C peuvent être remplacés par S, O ou $NR^4$,

$R^4$ étant choisi entre : H ; un reste alkyle en $C_1$ à $C_{10}$, saturé ou non saturé, ramifié ou non ramifié, substitué une ou plusieurs fois ou non substitué ;

$R^3$ est choisi entre H ; un reste alkyle en $C_1$ à $C_{10}$, saturé ou non saturé, ramifié ou non ramifié, substitué une ou plusieurs fois ou non substitué ; un reste cycloalkyle en $C_3$ à $C_8$, saturé ou non saturé, non substitué ou substitué une ou plusieurs fois ; un reste aryle ou un reste hétéroaryle, chacun non substitué ou substitué une ou plusieurs fois ; ou un reste aryle lié par l'intermédiaire d'un radical alkyle en $C_1$ à $C_3$ saturé ou non saturé, un reste cycloalkyle en $C_3$ à $C_8$ ou un reste hétéroaryle, chacun non substitué ou substitué une ou plusieurs fois ;

sous forme de son racémate ; de ses énantiomères, ses diastéréoisomères, en particulier de mélanges de ses énantiomères ou diastéréoisomères ou d'un énantiomère ou diastéréoisomère individuel ; sous forme de ses sels acides et basiques acceptables du point de vue physiologique, plus précisément ses sels formés avec des cations ou des bases ou bien avec des anions ou acides, ou sous forme des acides ou des bases libres ;

à l'exception de composés dans lesquels $R^1$, $R^2$ et $R^3$ représentent en même temps H ou bien $R^1$ et $R^2$ représentent en même temps $CH_3$ et $R^3$ correspond à l'hydrogène,

pour la préparation d'un médicament destiné au traitement de la douleur, en particulier d'une douleur neuropathique, chronique ou aiguë, de l'épilepsie et/ou de la migraine

ou bien

pour la préparation d'un médicament destiné au traitement de l'hyperalgésie et de l'allodynie, en particulier de l'hyperalgésie thermique, de l'hyperalgésie et l'allodynie mécaniques et de l'allodynie liée au froid, ou de douleur inflammatoire ou post-opératoire,

ou bien

pour la préparation d'un médicament destiné au traitement de bouffées de chaleur, de troubles post-ménopausiques, de la sclérose latérale amyotrophique (ALS), de l'algodystrophie sympathique réflexe (RSD), de la paralysie spastique, du Restless Leg Syndrom, du nystagmus acquis ; de troubles psychiatriques et neuropathologiques tels que troubles bipolaires, anxiété, crises de peur panique, variations de l'humeur, psychose maniaque, dépressions, psychose maniaco-dépressive ; de la neuropathie diabétique douloureuse, de symptômes et douleurs liés à la sclérose en plaques ou à la maladie de Parkinson, de maladies neurodégénératives telles que la maladie d'Alzheimer, la maladie de Huntington, la maladie de Parkinson et l'épilepsie ; d'une lésion gastro-intestinale ; de la douleur érythromélalgique ou post-poliomyélitique, de la névralgie faciale ou post-herpétique ; ou comme anticonvulsif, analgésique ou anxiolytique.

**2.** Utilisation suivant la revendication 1, **caractérisée en ce qu'**on utilise un thio-aminoacide de formule I, dans laquelle

$R^1$ et $R^2$ sont tous deux choisis, indépendamment l'un de l'autre, entre un reste alkyle en $C_1$ à $C_{10}$, ramifié

ou non ramifié, saturé ou non saturé, non substitué ou substitué une ou plusieurs fois ; un reste benzyle, aryle, cycloalkyle en $C_3$ à $C_8$ ou hétéroaryle, chacun non substitué ou substitué une ou plusieurs fois ;

ou bien

$R^1$ et $R^2$ forment ensemble un noyau $(CH_2)_{3-6}$, saturé ou non saturé, substitué ou non substitué, dans lequel 0 à 2 atomes de carbone peuvent être remplacés par S, O ou $NR^4$.

**3.** Utilisation suivant l'une des revendications 1 ou 2, **caractérisée en ce qu'**on utilise un thio-aminoacide de formule I, dans laquelle

$R^1$ et $R^2$ sont tous deux choisis, indépendamment l'un de l'autre, entre H ; un reste alkyle en $C_1$ à $C_{10}$, ramifié ou non ramifié, saturé ou non saturé, non substitué ou substitué une ou plusieurs fois ; un reste phényle ou un reste thiophényle, chacun non substitué ou substitué une fois ; ou un reste cycloalkyle en $C_3$ à $C_8$, non substitué ou substitué,

ou bien

$R^1$ et $R^2$ forment ensemble un noyau $(CH_2)_{3-6}$, substitué ou non substitué, dans lequel 0 ou 1 atome de carbone peut être remplacé par S, O ou $NR^4$.

**4.** Utilisation suivant la revendication 3, **caractérisée en ce qu'**on utilise un thio-aminoacide de formule I, dans laquelle

l'un des restes $R^1$ et $R^2$ est un reste alkyle en $C_1$ ou $C_2$, non substitué ou substitué une ou plusieurs fois ; ou un reste phényle, thiophényle, chacun non substitué ou substitué une fois ; ou un reste cycloalkyle en $C_3$ à $C_8$ non substitué ou substitué une fois ; et l'autre des restes $R^1$ et $R^2$ désigne un reste alkyle en $C_2$ à $C_{10}$, ramifié ou non ramifié, saturé ou non saturé, non substitué ou substitué une ou plusieurs fois ; ou un reste phényle ou thiophényle, chacun non substitué ou substitué une fois ; ou des restes cycloalkyle en $C_3$ à $C_8$ chacun non substitué ou substitué une fois ;

ou bien

$R^1$ et $R^2$ forment ensemble un reste cyclopropyle, cyclobutyle, cyclopentyle, cyclohexyle ou cycloheptyle, chacun non substitué ou substitué une fois, un atome de carbone du noyau étant éventuellement remplacé par S.

**5.** Utilisation suivant l'une des revendications 3 ou 4, **caractérisée en ce qu'**on utilise un thio-aminoacide de formule I dans laquelle

$R^1$ et $R^2$ sont tous deux choisis, indépendamment l'un de l'autre, entre H ; un reste alkyle en $C_1$ à $C_{10}$, ramifié ou non ramifié, saturé ou non saturé, non substitué ou substitué une ou plusieurs fois ; un reste phényle ou thiophényle, chacun non substitué ou substitué une fois avec $OCH_3$, $CH_3$, OH, SH, $CF_3$, F, Cl, Br ou I ; ou un reste cycloalkyle en $C_3$ à $C_8$ non substitué ou substitué ;

ou bien

l'un des restes $R^1$ et $R^2$ est un reste méthyle ou éthyle ; ou un reste phényle, thiophényle, chacun non substitué ou substitué une fois avec $OCH_3$, $CH_3$, OH, SH, $CF_3$, F, Cl, Br ou I ; ou un reste cycloalkyle en $C_3$ à $C_8$ non substitué ou substitué une fois ; et l'autre des restes $R^1$ et $R^2$ est un reste éthyle, n-propyle, isopropyle, n-butyle, isobutyle, tertiobutyle, pentyle, hexyle, heptyle ou octyle, ramifié ou non ramifié, saturé ou non saturé, non substitué ou substitué une ou plusieurs fois ; ou un reste phényle ou thiophényle, chacun non substitué ou substitué une fois avec $OCH_3$, $CH_3$, OH, SH, $CF_3$, F, Cl, Br ou I ; ou un reste cyclopropyle, cyclobutyle, cyclopentyle, cyclohexyle ou cycloheptyle ;

ou bien

$R^1$ et $R^2$ forment ensemble un reste cyclopropyle, cyclobutyle ou cyclopentyle, un atome de carbone du noyau étant éventuellement remplacé par S.

**6.** Utilisation suivant l'une des revendications 1 à 5, **caractérisée en ce qu'**on utilise un thio-aminoacide de formule I dans laquelle

$R^3$ est choisi entre H ; un reste alkyle en $C_1$ à $C_6$, saturé ou non saturé, ramifié ou non ramifié, substitué une ou plusieurs fois ou non substitué ; un reste phényle ou thiophényle non substitué ou substitué une fois ; ou un reste phényle lié par l'intermédiaire d'un radical saturé $CH_3$, non substitué ou substitué une fois.

**7.** Utilisation suivant la revendication 6, **caractérisée en ce qu'**on utilise un thio-aminoacide de formule I dans laquelle

$R^3$ est choisi entre un reste alkyle en $C_1$ à $C_6$, saturé, non ramifié et non substitué ; un reste phényle ou thiophényle, substitué une fois avec $OCH_3$, $CH_3$, OH, SH, $CF_3$, F, Cl, Br ou I ; ou un reste phényle lié par l'intermédiaire d'un radical saturé $CH_3$, substitué une fois avec $OCH_3$, $CH_3$, OH, SH, $CF_3$, F, Cl , Br ou I.

**8.** Utilisation suivant la revendication 7, **caractérisée en ce qu'**on utilise un thio-aminoacide de formule I dans laquelle

$R^3$ est choisi entre un reste méthyle, éthyle, propyle, n-propyle, isopropyle, butyle, n-butyle, isobutyle, tertiobutyle, pentyle ou hexyle.

9. Utilisation suivant l'une des revendications 1 à 8, **caractérisée en ce que**, pour le thio-aminoacide utilisé de formule I :

- lorsque l'un de $R^1$ ou $R^2$ représente l'hydrogène et $R^3$ est un reste benzyle ou H, l'autre de $R^1$ ou $R^2$ ne doit pas être un reste phényle,
- lorsque $R^1$ ou $R^2$ forment ensemble un reste cyclopentyle, $R^3$ ne doit pas représenter H,
- lorsque l'un de $R^1$ ou $R^2$ représente l'hydrogène et l'autre de $R^1$ ou $R^2$ représente un reste phényle, $R^3$ ne doit pas être un reste benzyle substitué ou non substitué, ou bien
- lorsque l'un de $R^1$ ou $R^2$ représente l'hydrogène et l'autre de $R^1$ ou $R^2$ représente un reste méthyle, $R^3$ ne doit pas représenter H.

10. Utilisation suivant l'une des revendications 1 à 9, **caractérisée en ce qu'**on utilise un thio-aminoacide choisi dans le groupe suivant :

- acide 2-amino-3-mercapto-3-méthyl-pentanoïque
- acide 2-amino-3-mercapto-3-méthyl-hexanoïque
- acide 2-amino-3-mercapto-3-méthyl-heptanoïque
- acide 2-amino-3-mercapto-3-méthyl-octanoïque
- acide 2-amino-3-mercapto-3-méthyl-nonanoïque
- acide 2-amino-3-mercapto-3-méthyl-décanoïque
- acide 2-amino-3-éthyl-3-mercapto-pentanoïque
- acide amino-(1-mercaptocyclopentyl)acétique
- acide amino-3-éthyl-3-mercapto-hexanoïque
- acide 2-amino-3-mercapto-3-méthyl-décanoïque
- acide 2-amino-3-mercapto-3-méthyl-nonanoïque
- acide 2-amino-3-mercapto-3-méthyl-octanoïque
- acide 2-amino-3-éthylsulfanyl-3-méthyl-octanoique
- acide 2-amino-3-benzylsulfanyl-3-méthyl-octanoïque
- acide 2-amino-3-mercapto-3-propyl-3-hexanoique
- acide amino-(1-mercaptocycloheptyl)acétique
- acide 2-amino-3-mercapto-3-propyl-3-hexanoïque
- acide amino-(1-mercaptocycloheptyl)acétique
- acide 2-amino-3-éthylsulfanyl-3-méthyl-nonanoïque
- acide 2-amino-3-méthyl-3-propylsulfanyl-nonanoïque
- acide 2-amino-3-hexylsulfanyl-3-méthyl-nonanoïque
- acide 2-amino-3-benzylsulfanyl-3-méthyl-nonanoïque
- acide 2-amino-3-benzylsulfanyl-3-méthyl-décanoïque
- acide 2-amino-3-éthylsulfanyl-3-méthyl-décanoïque
- acide 2-amino-3-cyclopropyl-3-(4-fluorophényl)-3-mercapto-propanoïque
- acide 2-amino-3-cyclopropyl-3-mercapto-butanoïque
- acide 2-amino-3-cyclobutyl-3-mercapto-butanoïque
- acide 2-amino-3-cyclohexyl-3-mercapto-butanoïque
- acide 2-amino-3-mercapto-3-thiophène-2-yl-butanoïque
- acide 2-amino-3-éthyl-3-mercapto-heptanoïque
- acide amino-(1-mercaptocyclohexyl)-éthanoïque
- acide amino-(1-mercapto-3-méthylcyclohexyl)-éthanoïque
- acide amino-(1-mercapto-2-méthylcyclohexyl)-éthanoique
- acide amino-(1-mercapto-4-méthylcyclohexyl)-éthanoïque
- acide amino-(4-mercaptotétrahydrothiopyranne-4-yl)-éthanoïque
- acide 2-amino-3-mercapto-3,4-diméthyl-pentanoïque
- acide 2-amino-3-mercapto-3,4-diméthyl-hexanoïque,

sous forme de son racémate ; de ses énantiomères, ses diastéréoisomères, de mélanges de ses énantiomères ou diastéréoisomères ou d'un énantiomère ou diastéréoisomère individuel ; sous forme de ses sels acides et basiques acceptables du point de vue physiologique, plus précisément ses sels formés avec des cations ou

des bases ou bien avec des anions ou des acides ou sous forme des acides ou des bases libres.

**11.** Utilisation de composés suivant la revendication 10, **caractérisée en ce que** les composés se présentent sous forme du chlorhydrate.

**12.** Utilisation suivant l'une des revendications 1 à 11, **caractérisée en ce qu'**au moins un thio-aminoacide utilisé se présente sous forme de diastéréoisomère et/ou d'énantiomère pur, sous forme de racémate ou sous forme de mélange non équimolaire ou équimolaire des diastéréoisomères et/ou des énantiomères.

**13.** β-thio-α-aminoacide de formule générale I

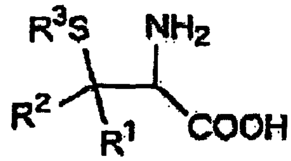

I

dans laquelle

l'un des restes $R^1$ et $R^2$ désigne un reste alkyle en $C_1$ à $C_6$, saturé ou non saturé, ramifié ou non ramifié, substitué une ou plusieurs fois ou non substitué ; et l'autre des restes $R^1$ et $R^2$ désigne un reste alkyle en $C_3$ à $C_{10}$, saturé ou non saturé, ramifié ou non ramifié, substitué une ou plusieurs fois avec F, Cl, Br, I, $NH_2$, SH ou OH ou non substitué ; ou un reste phényle, thiophényle ou cycloalkyle en $C_3$ à $C_8$, chacun non substitué ou substitué une ou plusieurs fois ;

et

$R^3$ est choisi entre H ; un reste alkyle en $C_1$ à $C_{10}$, saturé ou non saturé, ramifié ou non ramifié, substitué une ou plusieurs fois ou non substitué ; un reste cycloalkyle en $C_3$ à $C_8$, saturé ou non saturé, non substitué ou substitué une ou plusieurs fois ; un reste aryle ou hétéroaryle, chacun non substitué ou substitué une ou plusieurs fois ; ou un reste aryle lié par l'intermédiaire d'un reste alkyle en $C_1$ à $C_3$ saturé ou non saturé, un reste cycloalkyle en $C_3$ à $C_8$ ou un reste hétéroaryle, chacun non substitué ou substitué une ou plusieurs fois ;

sous forme de son racémate ; ses énantiomères, diastéréoisomères, en particulier de mélanges de ses énantiomères ou diastéréoisomères ou d'un énantiomère ou diastéréoisomère individuel ; sous forme de ses sels acides et basiques acceptables du point de vue physiologique, plus précisément ses sels formés avec des cations ou des bases ou bien avec des anions ou des acides ou sous forme des acides ou des bases libres.

**14.** Thio-aminoacide suivant la revendication 13, **caractérisé en ce que**

l'un des restes $R^1$ et $R^2$ désigne un reste alkyle en $C_1$ ou $C_2$ substitué une ou plusieurs fois ou non substitué, et l'autre des restes $R^1$ et $R^2$ désigne un reste alkyle en $C_3$ à $C_{10}$ saturé ou non saturé, ramifié ou non ramifié, substitué une ou plusieurs fois avec F, Cl, Br, I, $NH_2$, SH ou OH ou non substitué ; ou un reste phényle ou thiophényle, chacun non substitué ou substitué une fois ; ou un reste cyclopropyle, cyclobutyle, cyclopentyle, cyclohexyle ou cycloheptyle.

**15.** Thio-aminoacide suivant la revendication 14, **caractérisé en ce que**

l'un des restes $R^1$ et $R^2$ désigne un reste méthyle ou éthyle et l'autre des restes $R^1$ et $R^2$ est un reste alkyle en $C_3$ à $C_8$, saturé ou non saturé, ramifié ou non ramifié, substitué une ou plusieurs fois avec F, Cl, Br, I, $NH_2$, SH ou OH ou non substitué ; ou un reste phényle ou thiophényle, chacun non substitué ou substitué une fois avec $OCH_3$, $CH_3$, OH, SH, $CF_3$, F, Cl, Br ou I ; ou un reste cyclopropyle, cyclobutyle, cyclopentyle, cyclohexyle ou cycloheptyle.

**16.** Thio-aminoacide suivant la revendication 15, **caractérisé en ce que**

l'un des restes $R^1$ et $R^2$ désigne un reste méthyle ou éthyle et l'autre des restes $R^1$ et $R^2$ désigne un reste propyle, n-propyle, isopropyle, butyle, n-butyle, isobutyle, tertiobutyle, pentyle, hexyle, heptyle ou octyle.

**17.** Thio-aminoacide suivant l'une des revendications 13 à 16, **caractérisé en ce que**

$R^3$ est choisi entre H ; un reste alkyle en $C_1$ à $C_6$, saturé ou non saturé, ramifié ou non ramifié, substitué une ou plusieurs fois ou non substitué ; un reste phényle ou thiophényle, non substitué ou substitué une fois ; ou un

reste phényle lié par l'intermédiaire d'un radical saturé $CH_3$, non substitué ou substitué une fois.

**18.** Thio-aminoacide suivant la revendication 17, **caractérisé en ce que**
$R^3$ est choisi entre un reste alkyle en $C_1$ à $C_6$, saturé, non ramifié et non substitué ; un reste phényle ou thiophényle, substitué une fois avec $OCH_3$, $CH_3$, OH, SH, $CF_3$, F, Cl, Br ou I ; ou un reste phényle lié par l'intermédiaire d'un radical saturé $CH_3$, substitué une fois avec $OCH_3$, $CH_3$, OH, SH, $CF_3$, F, Cl, Br ou I.

**19.** Thio-aminoacide suivant la revendication 18, **caractérisé en ce que**
$R^3$ est choisi entre un reste méthyle, éthyle, propyle, n-propyle, isopropyle, butyle, n-butyle, isobutyle, tertiobutyle, pentyle ou hexyle.

**20.** Thio-aminoacide suivant l'une des revendications 13 à 19, **caractérisé en ce qu'**il est choisi dans le groupe suivant :

- acide 2-amino-3-mercapto-3-méthyl-hexanoique
- acide 2-amino-3-mercapto-3-méthyl-heptanoique
- acide 2-amino-3-mercapto-3-méthyl-octanoïque
- acide 2-amino-3-mercapto-3-méthyl-nonanoïque
- acide 2-amino-3-mercapto-3-méthyl-décanoïque
- acide amino-3-éthyl-3-mercapto-hexanoique
- acide 2-amino-3-mercapto-3-méthyl-décànoîque
- acide 2-amino-3-mercapto-3-méthyl-nonanoïque
- acide 2-amino-3-mercapto-3-méthyl-octanoïque
- acide 2-amino-3-éthylsulfanyl-3-méthyl-octanoïque
- acide 2-amino-3-benzylsulfanyl-3-méthyl-octanoïque
- acide 2-amino-3-mercapto-3-propyl-3-hexanoïque
- acide amino-(1-mercaptocycloheptyl)acétique
- acide 2-amino-3-mercapto-3-propyl-3-hexanoïque
- acide 2-amino-3-éthylsulfanyl-3-méthyl-nonanoïque
- acide 2-amino-3-méthyl-3-propylsulfanyl-nonanoïque
- acide 2-amino-3-hexylsulfanyl-3-méthyl-nonanoïque
- acide 2-amino-3-benzylsulfanyl-3-méthyl-nonanoique
- acide 2-amino-3-benzylsulfanyl-3-méthyl-décanoïque
- acide 2-amino-3-éthylsulfanyl-3-méthyl-décanoïque
- acide 2-amino-3-cyclopropyl-3-mercapto-butanoïque
- acide 2-amino-3-cyclobutyl-3-mercapto-butanoïque
- acide 2-amino-3-cyclohexyl-3-mercapto-butanoïque
- acide 2-amino-3-mercapto-3-thiophène-2-yl-butanoïque
- acide 2-amino-3-éthyl-3-mercapto-heptanoïque
- acide 2-amino-3-mercapto-3,4-diméthyl-pentanoïque
- acide 2-amino-3-mercapto-3,4-diméthyl-hexanoïque,

sous forme de son racémate ; de ses énantiomères, ses diastéréoisomères, de mélanges de ses énantiomères ou diastéréoisomères, ou d'un énantiomère ou diastéréoisomère individuel ; sous forme de ses sels acides ou basiques acceptables du point de vue physiologique, plus précisément ses sels formés avec des cations ou des bases ou avec des anions ou des acides, ou sous forme des acides ou des bases libres.

**21.** Thio-aminoacides suivant la revendication 20, **caractérisés en ce que** les composés se présentent sous la forme du chlorhydrate.

**22.** Médicament contenant au moins un thio-aminoacide suivant l'une des revendications 13 à 21 ainsi que, le cas échéant, des additifs et/ou des substances auxiliaires convenables et/ou éventuellement d'autres substances actives.

**23.** Médicament suivant la revendication 22, **caractérisé en ce qu'**un thio-aminonacide qu'il contient, selon l'une des revendications 13 à 21, est présent sous forme de diastéréoisomère et/ou d'énantiomère pur, sous forme de racémate ou sous forme d'un mélange non équimolaire ou équimolaire des diastéréoisomères et/ou des énantiomères.

**24.** Utilisation d'un thio-aminoacide suivant l'une des revendications 13 à 21,

pour la préparation d'un médicament destiné au traitement de la douleur, en particulier d'une douleur neuropathique, chronique ou aiguë, de l'épilepsie et/ou de la migraine

ou

pour la préparation d'un médicament destiné au traitement de l'hyperalgésie et de l'allodynie, en particulier de l'hyperalgésie thermique, de l'hyperalgésie et l'allodynie mécaniques et de l'allodynie liée au froid,

où d'une douleur inflammatoire ou post-opératoire,

ou

pour la préparation d'un médicament destiné au traitement de bouffées de chaleur, de troubles post-ménopausiques, de la sclérose latérale amyotrophique (ALS), de l'algodystrophie sympathique réflexe (RSD), de la paralysie spastique, du Restless Leg Syndrom, du nystagmus acquis ; de troubles psychiatriques et neuropathologiques tels que troubles bipolaires, anxiété, crises de peur panique, variations de l'humeur, psychose maniaque, dépressions, psychose maniaco-dépressive ; de la neuropathie diabétique douloureuse, de symptômes et douleurs liés à la sclérose en plaques ou à la maladie de Parkinson, de maladies neurodégénératives telles que la maladie d'Alzheimer, la maladie de Huntington, la maladie de Parkinson et l'épilepsie ; d'une lésion gastro-intestinale ; de la douleur érythromélalgique ou post-poliomyélitique, de névralgie faciale ou post-herpétique ; ou comme anticonvulsif, analgésique ou anxiolytique.

**25.** Procédé de production d'un thio-aminoacide suivant l'une des revendications 13 à 21, comprenant les étapes suivantes :

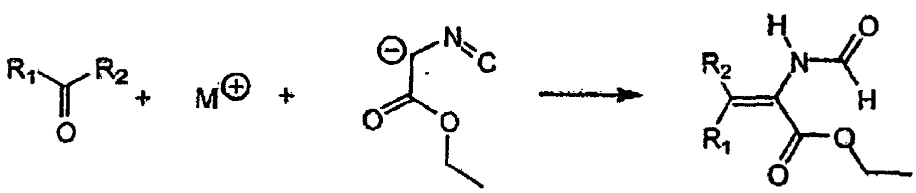

2

3

déprotonation de l'ester éthylique d'acide isocyanacétique avec des bases, avantageusement le butyl-lithium, l'hydroxyde de sodium ou le tertiobutylate de potassium, suivie de la réaction avec des cétones de formule générale 2 dans le tétrahydrofuranne conduisant à des esters éthyliques d'acides (E,Z)-2-formylamino-acryliques de formule générale 3,

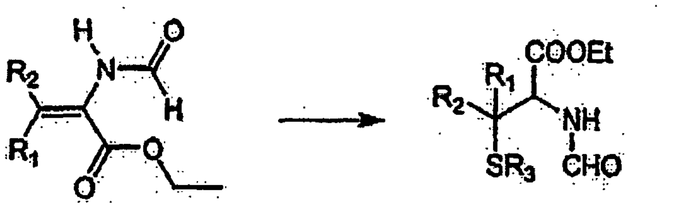

3

4

réaction d'esters éthyliques d'acides (E,Z)-2-formylamino-acryliques de formule générale 3 avec $P_4S_{10}$ dans le toluène ou avec des mercaptans de formule générale $R_3SH$ en présence de butyl-lithium dans le toluène, ce qui mène à un formylamino-éthylester de formule générale 4,

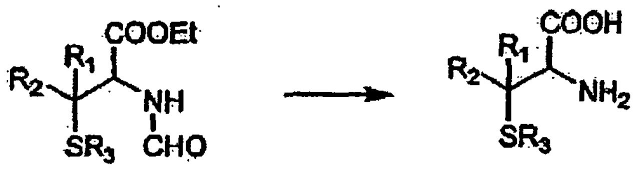

4 1

réaction des formylamino-éthylesters de formule générale 4 avec un acide, avantageusement l'acide chlorhydrique, ce qui mène aux thio-aminoacides de formule générale 1 ou I selon l'une des revendications 12 à 19, la réaction étant éventuellement suivie de, ou interrompue par, la séparation des diastéréoisomères au stade opportun par CLHP, chromatographie sur colonne ou cristallisation, ou bien suivie de la séparation des énantiomères par CLHP, chromatographie sur colonne ou cristallisation,

$R^1$ à $R^3$ ayant la définition selon la revendication 12 ou correspondant à un reste similaire protégé par un groupe protecteur convenable.